# EUROPÄISCHE PATENTSCHRIFT

(11) **EP 2 511 369 B1**
(45) Veröffentlichungstag und Bekanntmachung des Hinweises auf die Patenterteilung: **23.10.2013**
(21) Anmeldenummer: 12176321.3
(22) Anmeldetag: 19.08.2008
(51) Int. Cl.: C12N 9/80, C12N 15/62, C07K 14/005

(54) **Proteasestabile Zellwand lysierende Enzyme**
Protease-stable, cell wall-lysing enzymes
Enzyme lysant les parois cellulaires, résistant aux protéases

(30) Priorität: 22.08.2007 US 957351 P; 21.12.2007 DE 102007061929; 14.05.2008 DE 102008023448; 28.02.2008 US 32211 P; 22.08.2007 EP 07114785; 28.02.2008 EP 08152096
(43) Veröffentlichungstag der Anmeldung: 17.10.2012
(62) Teilanmeldung aus: 08801201.8
(73) Patentinhaber: Biomerieux S.A., 69280 Marcy l'Etoile (FR); Hyglos Invest GmbH, 82347 Bernried (DE)
(72) Erfinder: Grallert, Holger, 82362 Deutenhausen / Weilheim (DE); Forchheim, Michael, 93051 Regensburg (DE)
(74) Vertreter: Dehmel, Albrecht

(56) Entgegenhaltungen:
- SAO-JOSE CARLOS ET AL: "The N-terminal region of the Oenococcus oeni bacteriophage fOg44 lysin behaves as a bona fide signal peptide in Escherichia coli and as a cis-inhibitory element, preventing lytic activity on oenococcal cells", JOURNAL OF BACTERIOLOGY, Bd. 182, Nr. 20, Oktober 2000 (2000-10), Seiten 5823-5831, XP002506874, ISSN: 0021-9193
- DATABASE UniProt [Online] EBI; 1. Juni 2002 (2002-06-01), IANDOLO ET AL.: "Amidase", XP002477602, Database accession no. Q8SDS7_BPPHA
- DIAZ E ET AL: "CHIMERIC PHAGE-BACTERIAL ENZYMES A CLUE TO THE MODULAR EVOLUTION OF GENES", PROCEEDINGS OF THE NATIONAL ACADEMY OF SCIENCES OF THE UNITED STATES OF AMERICA, Bd. 87, Nr. 20, 1990, Seiten 8125-8129, XP002477600, ISSN: 0027-8424
- DATABASE UniProt [Online] EBI; 1. Dezember 2001 (2001-12-01), KURODA ET AL.: "Truncated Amidase", XP002477604, Database accession no. Q931M6_STAAM
- DATABASE UniProt [Online] ebi; 26. Juni 2007 (2007-06-26), COPELAND ET AL.: "SH3, type-5 domain protein.", XP002477605, Database accession no. A5IUB2_STAA9
- DATABASE Geneseq [Online] Derwent; 11. März 2004 (2004-03-11), MATSIZAKI ET AL.: "Staphylococcus aureus related bacteriophage protein #7.", XP002477606, Database accession no. ADN01958

## Beschreibung

Die vorliegende Erfindung betrifft eine Polypeptidvariante des Polypeptids gemäß SEQ ID NO: 1, wobei die Polypeptidvariante ausgewählt ist aus der Gruppe bestehend aus: SEQ ID NO: 2, 3, 11, 12 und 13. Die Erfindung betrifft ferner Nukleinsäuren mit einer Sequenz codierend für die erfindungsgemäßen Polypeptide.

Pathogene Bakterien treten an vielen Stellen auf und verursachen enorme gesundheitliche Probleme durch Infektionen sowie hohe wirtschaftliche Kosten z.B. auch durch unerwünschten Bakterienbefall in der Nahrungsmittel-, Kosmetik- oder Umweltindustrie. Im Gesundheitswesen nehmen die Probleme aufgrund Antibiotika-resistenter Keime immer mehr zu, so dass händeringend nach Alternativen zu Antibiotika gesucht wird. In der Lebensmittel-und Kosmetikindustrie wird zunehmend versucht, ohne die klassischen Konservierungsmittel auszukommen, die in der Bevölkerung immer weniger Akzeptanz finden. Als Lösung für diese Probleme bietet sich der Einsatz von Zellwand lysierenden Enzymen an, die auf natürliche Weise das Wachstum der unerwünschten Bakterien unterbinden und vorhandene Keime abtöten.

Beispiele von Zellwand lysierenden Enzymen sind Endolysine, die aus Bakteriophagen, isoliert wurden. Bakteriophagen benutzen diese Enzyme am Ende ihres Vermehrungszyklus, um die innerhalb der Bakterienzelle gebildeten Bakteriophagen freizusetzen. Die Bakterienhülle wird dabei lysiert und das Wirtsbakterium auf diese Weise zerstört. Ein weiteres Beispiel sind Enzyme, die der Bakteriophage am Anfang seines Vermehrungzyklus benötigt und in der Regel in Bakteriophagenschwanzproteinen lokalisiert sind. Diese Enzyme werden für den Aufbruch der Bakterienwand bei der Bakterieninfektion benötigt. Ein drittes Beispiel sind Enzyme, die eine ähnliche Funktion und häufig auch eine sequenzielle Ähnlichkeit zu den Endolysinen aufweisen. Diese Enzyme sind die von Bakterien unter bestimmten Umständen gebildeten Autolysine, die zur Selbstlyse der Bakterien führen. Ein viertes Beispiel sind Enzyme, die ebenfalls von Bakterien gebildet werden und als Bakteriocine bekannt sind. Diese vier Gruppen von Zellwand lysierenden Enzyme werden bereits technisch eingesetzt und in Zukunft wohl noch an Bedeutung gewinnen.

Ein Anwendungsgebiet ist der medizinische Einsatz in der Prävention und Therapie sowie die Diagnostik von Bakterieninfektionen bei Mensch und Tier. Ein weiteres Anwendungsgebiet ist die Anwendung in der Lebensmittel-, Umwelt- und Kosmetikindustrie zur Verhinderung eines unerwünschten Bakterienwachstums und zur Abtötung der Keime beispielsweise durch Desinfektion sowie der Nachweis von Bakterien in Lebensmittel-, Umwelt- und Kosmetikproben.

Praktisch alle Anwendungen in denen Bakterien lysierende Enzyme verwendet werden stellen hohe Anforderungen an die Stabilität der eingesetzten Enzyme, damit sich ihr Einsatz wirtschaftlich lohnt. In der US 6,432,444 B1 wird zur Stabilisierung der Zellwand lysierenden Enzyme vorgeschlagen, z.B. einen stabilisierenden Puffer zu verwenden, um die optimale Enzymaktivität zu gewährleisten. Weiterhin wird der Zusatz von stabilisierenden Substanzen wie Reduktionsmittel, Metallchelatoren, Immuglobulinen, spezifischen Puffersalzen, physiologischen pH-Werten, Konservierungsmittel oder milden Detergentien vorgeschlagen.

Generell wird die Stabilität von Proteinen häufig durch vorhandene Proteasen verringert. Strategien zur Erhöhung der Stabilität von Proteinen gegenüber einem Proteaseabbau sind z.B. der Zusatz von Metallchelatoren, um Proteasen zu hemmen, die für ihre Aktivität Metallionen benötigen oder der Zusatz von speziellen Proteaseinhibitoren, wie sie zum Beispiel für Serinproteasen kommerziell erhältlich sind. Proteaseinhibitoren können zur Stabilität von Zellwand lysierenden Enzymen jedoch nur bedingt eingesetzt werden, weil sie auch andere essentielle Komponenten am Wirkort stören. Darüber hinaus sind spezifische Inhibitoren auch sehr teuer. Auch die Auswahl anderer Umgebungsbedingungen, in denen Proteasen weniger aktiv sind, ist in der Regel nicht möglich, da die Zellwand lysierenden Enzyme ganz ähnliche Bedingungen für ihre Aktivität brauchen wie die Proteasen. So arbeiten Zellwand lysierende Enzyme in einer wässrigen Umgebung und unter moderaten pH-Werten am besten, unter denen auch die Proteaseaktivität am größten ist. Die bekannten Stabilisierungsansätze sind für den Einsatz bei Zellwand lysierenden Enzymen nicht geeignet.

Daher liegt der vorliegenden Erfindung die Aufgabe zu Grunde, stabile Zellwand lysierende Enzyme zur Verfügung zu stellen.

Die Aufgabe wird durch den in den Patentansprüchen definierten Gegenstand gelöst.

Die nachfolgenden Abbildungen dienen der Erläuterung der Erfindung.

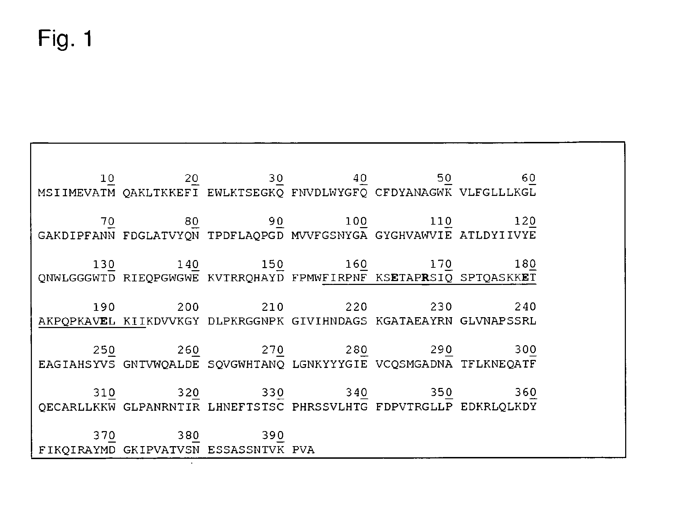

Figur 1 zeigt die Wildtyp-Aminosäuresequenz der CHAP-Domäne (Aminosäure 1-154), der Linkerregion (unterstrichen, Aminosäure 155-193) und der Amidase-Domäne (Aminosäure 194-393) eines Endolysins aus PlyPitti26. Potentielle Schnittstellen für V8 Protease, die nach dem Aminosäurerest E schneidet, sind fett hervorgehoben und befinden sich an den Aminosäurepositionen 163, 179 und 189. Die Thrombinschnittstelle ist grau unterlegt und befindet sich an Position R167.

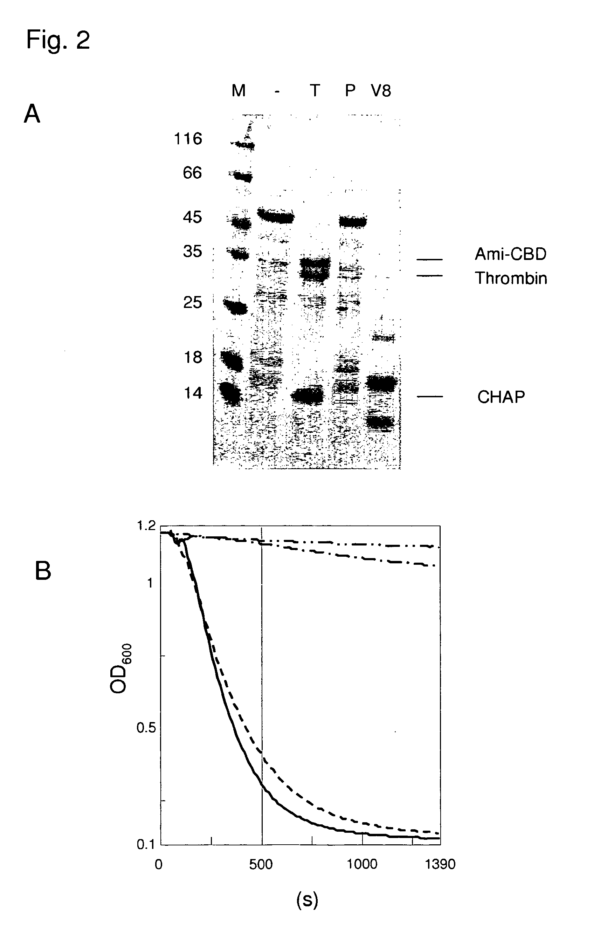

Figur 2 zeigt das Ergebnis eines Proteaseverdaus und anschließendem Aktivitätstest von nicht modifiziertem PlyPitti26 mit den Proteasen Thrombin und V8 Protease. Figur 2A zeigt ein SDS-Polyacrylamidgel mit Proben von nicht modifizeirtem PlyPitti26 nach einen Verdau mit Thrombin (T), V8 Protease (V8) und einem Kontrollverdau mit Plasmin (P), für das keine Schnittstelle existiert. Unverdautes PlyPitti26 ist mit "-" gekennzeichnet. M bezeichnet Molekulargewichtsstandards mit den am Rand angegebenen Proteingrößen in kDa. Am rechten Rand angegeben sind die Positionen der Banden für das Fragment N-terminal von der Thrombinschnittstelle (CHAP-Domäne), das Fragment C-terminal von der Schnittstelle (Ami-CBD) und die Bande vom zugesetzten Thrombin. Figur 2B zeigt das Ergebnis eines Flüssiglysetests zur Bestimmung der spezifischen Aktivitäten von verdautem und nicht verdautem nicht modifiziertem PlyPitti26. Die Bakterienzelllyse wird über eine Lichtstreuungsmessung im Photometer verfolgt. Es sind die Lysekurven für unverdautes PlyPitti26 (____), Plasmin verdautes (-----) sowie mit Thrombin (_._.) oder V8 Protease (_.._..) verdautes Endolysin dargestellt.

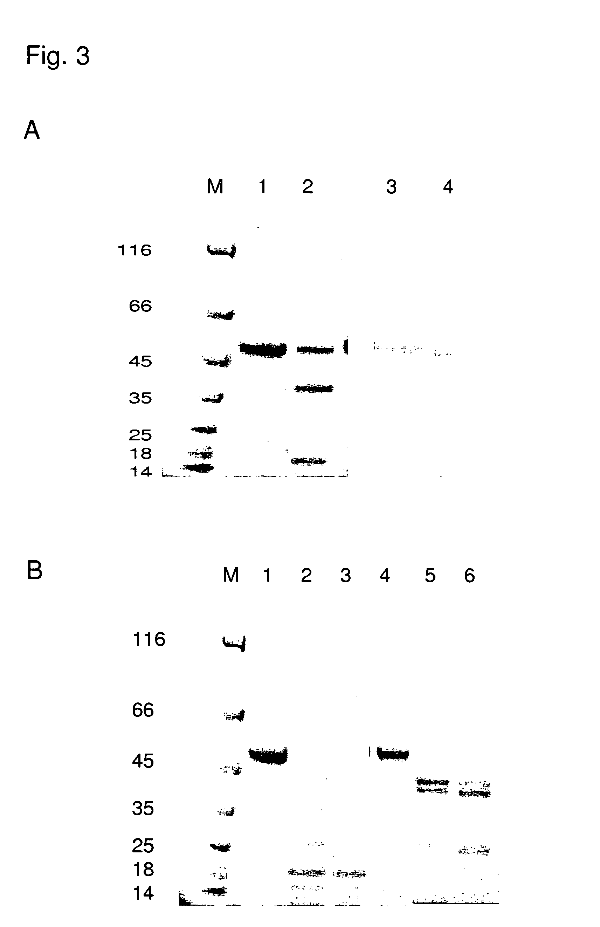

Figur 3 zeigt das Ergebnis eines Proteaseverdaus von modifiziertem und nicht modifiziertem CHAP-AmiPitti26-CBDUSA. Figur 3A zeigt das Ergebnis eines Thrombinverdaus des nicht modifizierten CHAP-AmiPitti26-CBDUSA und eines modifizierten *Staphylococcus* Endolysins CHAP-AmiPitti26-CBDUSA (Mutante 4 auf Tabelle 3). Das nicht modifizierte CHAP-AmiPitti26-CBDUSA besitzt eine singuläre Thrombinschnittstelle zwischen der CHAP- und der Amidase-Domäne an Position R167, das modifizierte Polypeptid einen Austausch von R nach A an Position 167. Die Figur 3A zeigt ein SDS-Polyacrylamidgel der beiden Enzymvarianten vor und nach dem Thrombinverdau: M - Molekulargewichtsmarker (Molekulargewichte in kDa am Rand angegeben); 1- nicht modifiziertes CHAP-AmiPitti26-CBDUSA ohne Thrombin; 2 - nicht modifiziertes CHAP-AmiPitti26-CBDUSA nach Thrombinverdau; 3 - modifiziertes CHAP-AmiPitti26-CBDUSA ohne Thrombin; 4-modifiziertes CHAP-AmiPitti26-CBDUSA nach Thrombinzugabe.
Figur 3B zeigt das Ergebnis eines V8 Protease Verdaus des nicht modifizierten CHAP-AmiPitti26-CBDUSA und eines modifizierten *Staphylococcus* Endolysins CHAP-AmiPitti26-CBDUSA (Mutante 4 auf Tabelle 3). Das nicht modifizierte CHAP-AmiPitti26-CBDUSA besitzt eine V8 Protease Schnittstelle zwischen der CHAP- und der Amidase-Domäne an Position E163, das modifizierte Polypeptid einen Austausch von E nach A an Position 163. Die Figur 3B zeigt ein SDS-Polyacrylamidgel der beiden Enzymvarianten vor und nach dem Thrombinverdau: M - Molekulargewichtsmarker (Molekulargewichte in kDa am Rand angegeben); 1- nicht modifiziertes CHAP-AmiPitti26-CBDUSA ohne V8 Protease; 2 - nicht modifiziertes CHAP-AmiPitti26-CBDUSA 15 min V8 Protease Verdau; 3 - nicht modifiziertes CHAP-AmiPitti26-CBDUSA 60 min V8 Protease Verdau; 4 - modifiziertes CHAP-AmiPitti26-CBDUSA ohne V8 Protease; 5 - modifiziertes CHAP-AmiPitti26-CBDUSA 15 min V8 Protease Verdau; 6 - modifiziertes CHAP-AmiPitti26-CBDUSA 60 min V8 Protease Verdau.

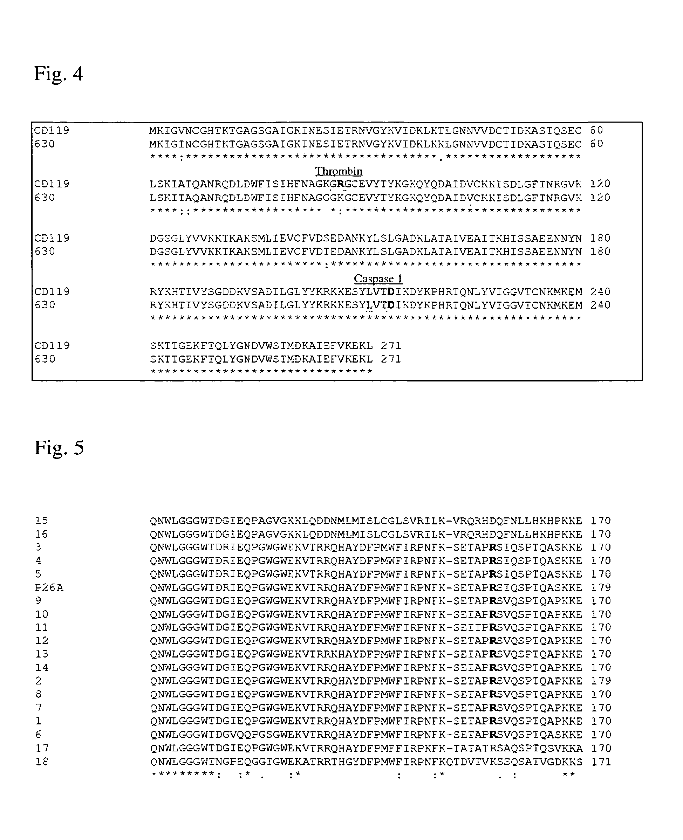

Figur 4 zeigt einen Aminosäuresequenzvergleich des modifizierten Endolysins aus dem Cl. difficile Phagen Φ CD 119 (CD 119) mit dem modifizierten Endolysin aus dem Cl. difficile Phagen Φ 630. Die im Φ CD 119 und Φ 630 Wildtyp vorhandene singuläre Caspase 1 Schnittstelle an Aminosäure D214 wurde zu E214 modifiziert, die im Φ 630 Wildtyp vorhandene singuläre Thrombinschnittstelle R87 zu K87 modifiziert.
Figur 5 zeigt einen Aminosäuresequenzvergleich der Region der Thrombinschnittstelle von verschiedenen *Staplaylococcus aureus* Endolysinen. Der fett hervorgehobene Aminosäurerest R ist Position R167 in der Referenzsequenz ply_pitti26, bezeichnet als P26A. Die weiteren aufgeführten Aminosäuresequenzen sind an R167 von ply_pitti26 ausgerichtget. Der Aminosäuresequenzvergleich wurde mit dem Programm BLAST durchgeführt; Altschul et al., 1990, J. Mol. Biol., 215, 403-410. Die Zahl rechts neben dem Aminosäuresequenzvergleich bezeichnet die Aminosäureposition der letzen Aminosäure im dargestellten Sequenzabschnitt des jeweiligen Endolysins. Die Zahlen am linken Rand stehen für verschiedene homologe Proteine aus dem Aminosäuresequenzvergleich. P26A steht für ply_pitti26, 15 ein Autolysin (N-Acetylmuramoyl-L-Alanin Amidase) aus *Staphylococcus aureus* (Datenbankzugangsnummer P24556), 16 steht für eine Amidase aus dem Bakteriophagen 80 alpha (Datenbankzugangsnummer AAB39699), 3 steht für das Endolysin des *Staphylococcus* Phagen phi MR11 (Datenbankzugangsnummer YP 001604156), 4 steht für ORF006 aus dem *Staphylococcus aureus* Phagen 88 (Datenbankzugangsnummer YP 240699), 5 steht für ORF21 aus dem *Staphylococcus aureus* Phagen 85 (Datenbankzugangsnummer YP 239752), 9 steht für die Amidase aus dem *Staphylococcus* Phagen 11 (Datenbankzugangsnummer NP803306), 10 steht für ORF007 aus dem *Staphylococcus* Phagen 52A (Datenbankzugangsnummer YP 240634), 11 steht für die N-Acetylmuramoyl-L-Alanin Amidase aus dem Stamm *Staphylococcus aureus subsp. aureus* JH9 (Datenbankzugangsnummer YP 001246457), 12 steht für die putative Zellwandhydrolase des Phagen phiMR25 (Datenbankzugangsnummer YP 001949866), 13 steht für ORF007 aus dem *Staphylococcus* Phagen 69 (Datenbankzugangsnummer YP 239596), 14 steht für ORF007 aus dem *Staphylococcus* Phagen 55 (Datenbankzugangsnummer YP 240484), 2 steht für die N-Acetylmuramoyl-L-Alanin Amidase aus dem Stamm *Staphylococcus aureus subsp. aureus* JH9 (Datenbankzugangsnummer YP 001245749), 8 steht für ORF007 aus dem *Staphylococcus* Phagen 29 (Datenbankzugangsnummer YP 240560), 7 steht für das Autolysin aus dem Stamm *Staphylococcus aureus subsp. aureus* NCTC 8325 (Datenbankzugangsnummer YP 500516), 1 steht für die Amidase aus dem *Staphylococcus* Phagen phiNM2 (Datenbankzugangsnummer ABF73160), 6 steht für eine Phagen-verwandte Amidase aus dem Stamm *Staphylococcus aureus* RF122 (Datenbankzugangsnummer YP 417165), 17 steht für ORF006 aus dem *Staphylococcus* Phagen 37 (Datenbankzugangsnummer YP 240103) und 18 steht für ORF007 aus dem *Staphylococcus* Phagen EW (Datenbankzugangsnummer YP 240182).

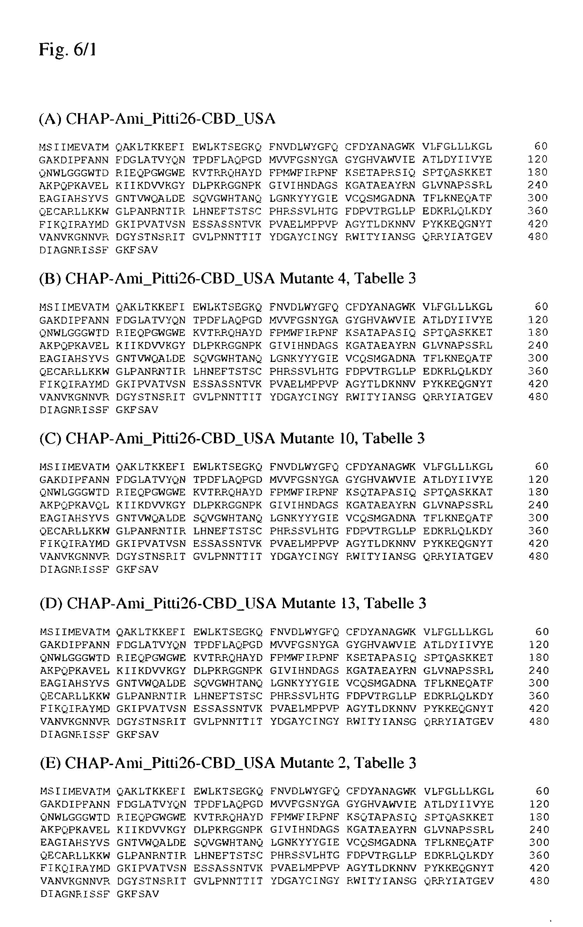

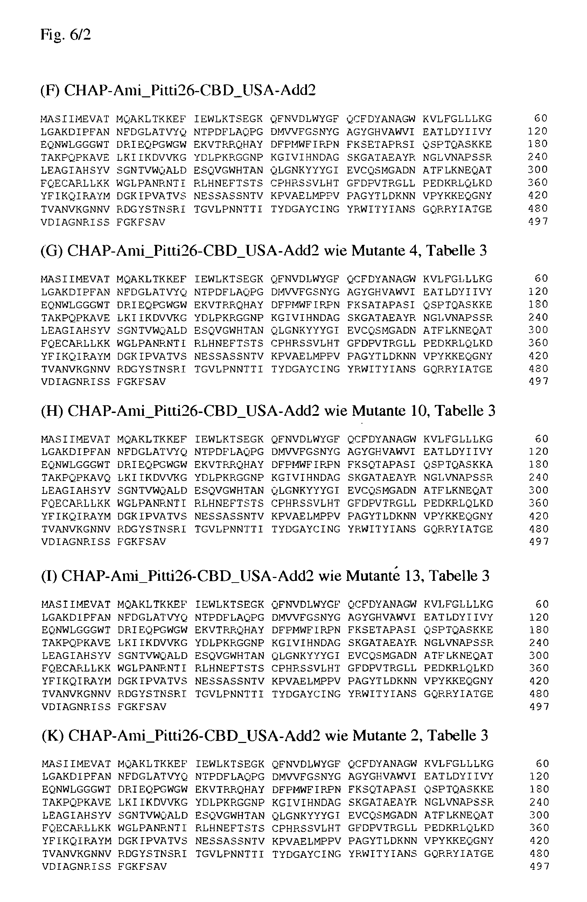

Figur 6 gibt Aminosäuresequenzen von modifiziertem und nicht modifiziertem CHAP-AmiPitti26-CBDUSA wieder. Figur 6A bis D zeigt die Aminosäuresequenz von CHAP-AmiPitti26-CBDUSA ohne Aminosäuresubstitutionen an Proteaseschnittstellen (A) und CHAP-AmiPitti26-CBDUSA mit Aminosäuresubstitutionen an Proteaseschnittstellen (B bis E). Figur 6F bis K zeigt die Aminosäuresequenz von CHAP-AmiPitti26-CBDUSA-Add2 ohne Aminosäuresubstitutionen an Proteaseschnittstellen und einem zusätzlichen Aminosäurerest an Position zwei (F) und CHAP-AmiPitti26-CBDUSA-Add2 mit Aminosäuresubstitutionen an Proteaseschnittstellen und einem zusätzlichen Aminosäurerest an Position zwei (G bis K).

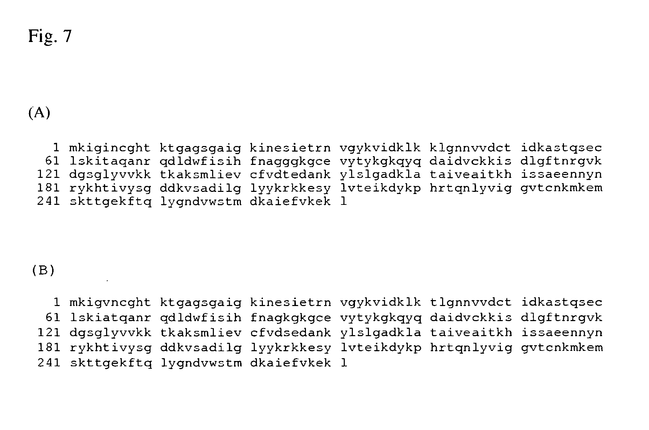

Figur 7A zeigt die Aminosäuresequenz des modifizieten Endolysins aus dem Cl. difficile Stamm 630 mit einem Austausch von D214 zu E214. Figur 7B zeigt die Aminosäuresequenz des modifizieten Cl. difficile Endolysins aus dem Phagen phi CD119 mit einem Austausch von R87 zu K87 und D214 zu E214.

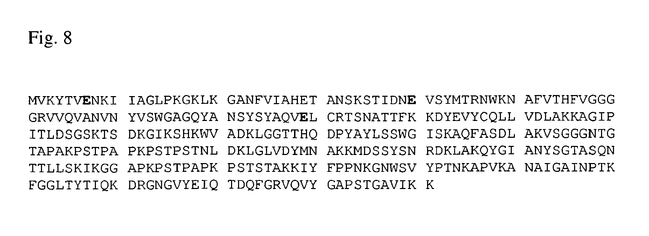

Figur 8 zeigt die Aminosäuresequenz des Endolysins Ply511. Die fett gedruckten Aminosäurepositionen E7, E40 und E89 können mit jedem anderen Aminosäurerest substituiert werden.

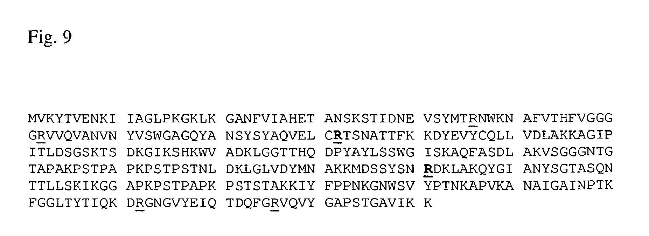

Figur 9 zeigt die Aminosäuresequenz des Endolysins Ply511. Die potentiellen Schnittstellen (R in P1 Position) für die Protease Clostripain sind unterstrichen. Die beiden experimentell ermittelten besonders sensitiven Schnittstellen (R92 und R221) sind unterstrichen und fett gedruckt.

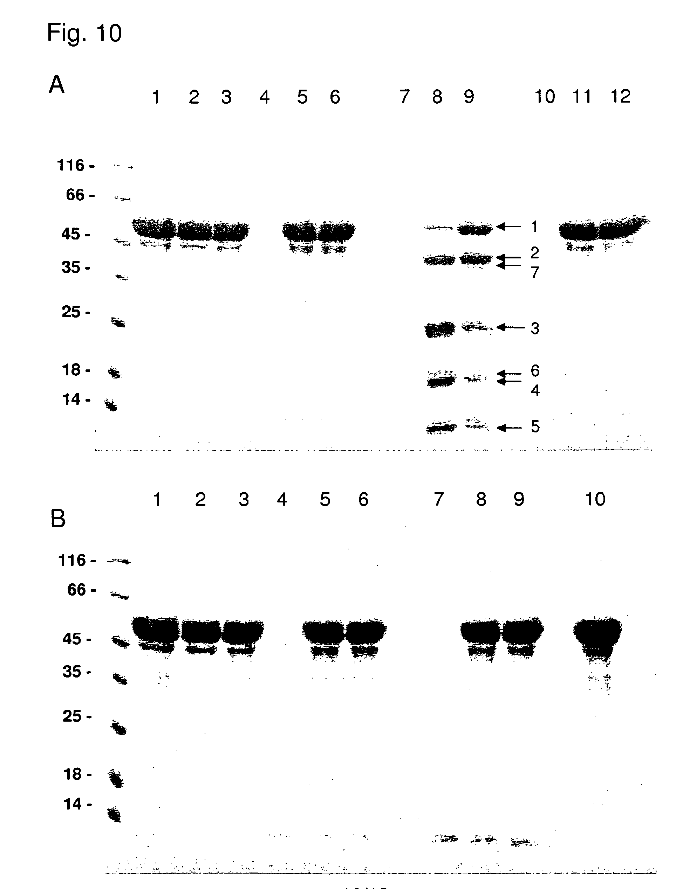

Figur 10 zeigt den Verdau des therapeutisch einsetzbaren Endolysins CHAP-Amipitti26_CBDUSA-Add2 (L56H, L57T, E164A, R168A, Y201H) durch in Organextrakten vorhandene Proteasen. Figur 10A zeigt ein SDS-Gel, bei dem die Proteinbanden nach Inkubation mit Leber-, Nieren- und Lungenextrakt aufgetrennt wurden. Links aufgetragen ist ein Molekulargewichtsstandard. Spuren 1 bis 3 zeigen Kontrollen (nur Endolysin, kein Organextrakt), die gar nicht (Spur 1), 18 h bei Raumtemperatur (Spur 2) oder 18 h bei 4 °C inkubiert (Spur 3) wurden. In den Spuren 4 bis 6 wurde Leberextrakt zugegeben, bei Spur 7 bis 9 Nierenextrakt und in den Spuren 10 bis 12 Lungenextrakt. Spuren 4, 7 und 10 enthalten nur Organextrakt, Spuren 5, 8 und 11 Organextrakt und Endolysin nach 18 h Inkubation bei Raumtemperatur, Spuren 6, 9 und 12 Organextrakt und Endolysin nach 18 h Inkubation bei 4 °C. Die Pfeile mit den Nummern 1 bis 7 neben Spur 9 markieren die Banden, die nach Transfer auf eine Blotmembran N-terminal ansequenziert wurden. Figur 10B zeigt ein SDS-Gel, bei dem die Proteinbanden nach Inkubation mit Herz- oder Milzextrakt aufgetrennt wurden. Links aufgetragen ist ein Molekulargewichtsstandard. Spuren 1 bis 3 sowie 10 zeigen Kontrollen (nur Endolysin, kein Organextrakt), die gar nicht (Spur 1 und 10), 18 h bei Raumtemperatur (Spur 2) oder 18 h bei 4 °C inkubiert (Spur 3) wurden. In den Spuren 4 bis 6 wurde Herzextrakt zugegeben, bei Spur 7 bis 9 Milzextrakt. Spuren 4 und 7 enthalten nur Organextrakt, Spuren 5 und 8 Organextrakt und Endolysin nach 18 h Inkubation bei Raumtemperatur, Spuren 6 und 9 Organextrakt und Endolysin nach 18 h Inkubation bei 4 °C.

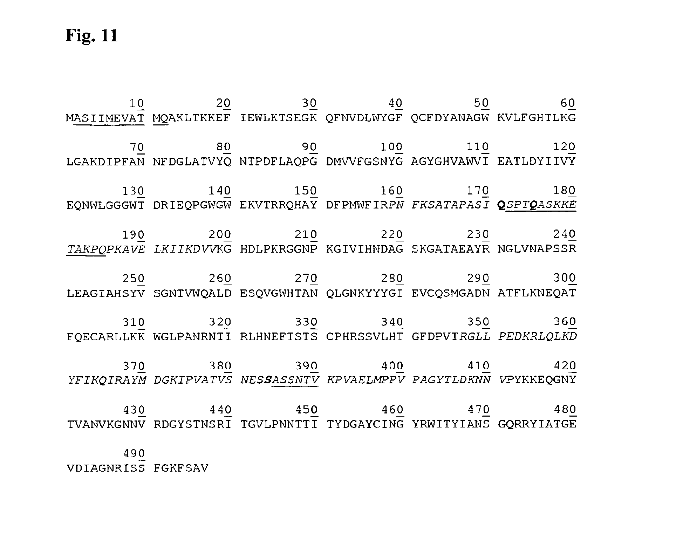

Fig. 11 zeigt die Sequenz des Endolysins CHAP-Amipitti26_CBDUSA-Add2 (L56H, L57T, E164A, R168A, Y201H) mit proteasesensitiven Bereichen. Die Schnittstellen (P1 Position), die sich aus der Inkubation mit Nierenextrakt ergaben, sind fett markiert. Aminosäuresequenzen, die sich bei der N-terminalen Sequenzierung der entstandenen Fragmente ergaben, sind unterstrichen. Aminosäurebereiche, die die Domänenlinker definieren, sind kursiv dargestellt. Dies ist ein Domänenlinkerbereich zwischen der CHAP und Amidase 2 Domäne (Aminosäuren 159 bis 198) sowie ein zweiter Linker zwischen der Amidase 2 Domäne und der CBD (Aminosäuren 347 bis 412).

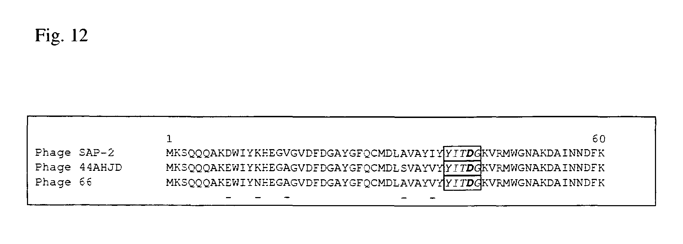

Fig. 12 zeigt einen Aminosäuresequenzvergleich der Region der Caspase I Schnittstelle von verschiedenen *Staphylococcus* Endolysinen. Es handelt sich um Amidasen aus den *Staphylococcus* Phagen SAP-2, 44AHJD und Phage 66. Die NCBI Datenbankzugangsnummern für die Proteine sind YP_001491539, 44AHJD_15 amidase und YP_239469. Die Position D42, nach der geschnitten wird ist fett hervorgehoben, die konservierte Erkennungssequenz für die Caspase I ist jeweils kursiv und umrandet dargestellt. Die 3 Proteine sind auf ihre gesamte Länge zu 89 bis 90 % identisch, nicht konservierte Aminosäuren für den Bereich der Aminosäuren 1 bis 60 sind mit "-" markiert. Der Aminosäuresequenzvergleich wurde mit dem Programm BLAST durchgeführt.

Der Begriff "Polypeptid" oder "Protein" wie hier verwendet bezeichnet Peptide aus mindestens acht Aminosäuren. Die Polypeptide können pharmakologisch oder immunologisch aktive Polypeptide oder für diagnostische Zwecke verwendete Polypeptide sein.

Der Begriff "Zellwand lysierende Enzyme" wie hier verwendet bezeichnet Enzyme, die in der Lage sind, die bakterielle Zellwand zumindest teilweise aufzubrechen oder so zu beschädigen, dass in der Folge eine Zelllyse oder zumindest ein bakteriostatischer Effekt auftritt. Insbesondere bezeichnet der Begriff Endolysine, Autolysine, Bakteriophagenschwanzproteine und Bakteriocine.

Der Begriff "Endolysin" wie hier verwendet bezeichnet Enzyme, die natürlicherweise von Bakteriophagen kodiert werden und die diese am Ende ihres Wirtszyklus herstellen, um die Wirtszelle zu lysieren und damit die in der Wirtszelle neu hergestellten Bakteriophagen freizusetzen. Endolysine sind aus mindestens einer enzymatisch aktiven Domäne (EAD) und einer nicht enzymatisch aktiven zellbindenden Domäne (CBD) aufgebaut. Die einzelnen EADs weisen unterschiedliche Aktivitäten auf, wobei Endolysine mindestens eine EAD ausgewählt aus der folgenden Gruppe aufweisen: N-Acetyl-Muramoyl-L-Alanin-Amidase (Amidase, z.B. Ami_2, Ami_5), (Endo)-Peptidase (z.B. CHAP, i.e. cysteine, histidine-dependent amidohydrolases/peptidases), Transglykosylase, Glykosylhydrolase, (N-Acetyl)-Muramidase (Lysozyme) und N-Acetyl-Glukosaminidase.

Der Begriff "Autolysin" wie hier verwendet bezeichnet bakterielle Peptidoglykanhydrolasen, die in bestimmten Situationen zu einer Selbstlyse der Bakterien führen. Funktionell und auch strukturell sind Autolysine und Endolysine häufig verwandt. Verschiedene Domänen aus Endolysinen und Autolysinen können häufig modular kombiniert werden und aktive Chimären bilden.

Der Begriff "Bakteriophagenschwanzprotein" wie hier verwendet bezeichnet Phagenstrukturproteine, die am Anfang des Replikationszyklus des Bakteriophagen bei der Infektion die Funktion erfüllen, an Rezeptoren auf der Bakterienoberfläche zu binden und dabei über ihre enzymatische Funktion Komponenten der Zelloberfläche lysieren. Die entsprechenden Bakteriophagenproteine müssen nicht immer im Phagenschwanz lokalisiert sein, sondern können bei Phagen ohne Schhwanz auch direkt am Phagenkopf sitzen.

Der Begriff "Bakteriocin" wie hier verwendet bezeichnet proteinogene Toxine, die von Bakterien abgesondert werden, um das Größenwachstum ähnlicher Bakteriengattungen zu inhibieren. Sie bestehen aus einer Zellwand bindenden Domäne, einer Translokationsdomäne und aus dem "killing factor". Die in diesem Zusammenhang enthaltene Gruppe der Bakteriocine greift dabei bakterielle Membranen an. Beispiele sind Colicin, Microcin, Nisin, Epidermin und Lantibiotika (lanthioninhaltige Antibiotika).

Der Begriff bakterielle "Zellwand" wie hier verwendet bezeichnet alle Komponenten, die die äußere Zellhülle der Bakterien aufbauen und so deren Unversehrtheit garantieren. Insbesondere ist darunter zu verstehen das Peptidoglykan, die äußere Membran der gramnegativen Bakterien mit dem Lipopolysaccharid, die Bakterienzellmembran, aber auch zusätzliche auf das Peptidoglykan aufgelagerte Schichten wie Kapseln, Schleime oder äußere Proteinschichten.

Der Begriff "Protease" wie hier verwendet bezeichnet ein Enzym, das in der Lage ist, Proteine und/oder Peptide hydrolytisch zu spalten.

Der Begriff "Domäne" wie hier verwendet bezeichnet einen Teilbereich einer Aminosäuresequenz, der funktionell und/oder strukturell definiert ist. Domänen können aufgrund von Homologien häufig sehr gut über entsprechende Suchprogramme vorhergesagt werden, die sich auf entsprechende Datenbanken mit konservierten Domänen stützen (z.B. Conserved Domain Database (CDD) an der NCBI (Marchler-Bauer et al., 2005, Nucleic Acids Res. 33, D192-6), Pfam (Finn et al., 2006, Nucleic Acids Research 34, D247-D251) oder SMART (Schultz et al., 1998, Proc. Natl. Acad. Sci. USA 95, 5857-5864, Letunic et al., 2006, Nucleic Acids Res 34, D257-D260).

Der Begriff "Wildtyp" wie hier verwendet bezeichnet die Aminosäuresequenz, wie sie in natürlicherweise vorkommenden Proteinen vorkommt und deren Sequenz nicht in Bezug auf Proteaseschnittstellen modifiziert wurde. Es kann sich dabei um Aminosäuresequenzen handeln so wie sie natürlicherweise in einem Bakteriophagen, Prophagen oder Bakterium vorkommen. Zusätzlich bezeichnet der Begriff alle in den Datenbanken vorkommenden Aminosäuresequenzen von Zellwand lysierenden Enzymen, die noch bestimmte Proteaseschnittstellen enthalten, auch wenn die Aminosäuresequenzen bereits gegenüber natürlich vorkommenden Proteinen verändert wurden. Im Zusammenhang mit Nukleotidsequenzen bezeichnet der Begriff Wildtyp auch die Nukleotidsequenzen, die für ein Polypeptid kodieren, die eine vorstehend beschriebene Aminosäuresequenz aufweisen.

Die vorliegende Erfindung betrifft ein modifiziertes Polypeptid mit der biologischen Aktivität Zellwände von Bakterien zu lysieren, wobei das Polypeptid keine Caspase-, Enterokinase-, Faktor Xa, Granzyme B, Staphylococcus Peptidase I (V8 Protease), Plasmin, Streptopain, Bacillolysin oder Thrombinschnittstelle enthält sowie weitere Proteaseschnittstellen, die spezifisch für das jeweilige Polypeptid durch Proteaseverdau mit anschließender Analyse der entstandenen Fragmente bestimmt wurden und anschließend auf die in der Erfindung beschriebene Weise modifiziert wurden. Vorzugsweise betrifft die vorliegende Erfindung erfindungsgemäße modifizierte Polypeptide, die gram positive Bakterien lysieren, wobei die Bakterien aus der Gruppe bestehend aus Clostridien, Bazillen, Listerien, Staphylokokken, Lactobazillen, Enterokokken, Aerokokken, Pediokokken, Streptokokken, Mycoplasmen und/oder Leuconostoc stammen können. Vorzugsweise ist das erfindungsgemäße Polypeptid ein Endolysin, ein Bakteriophagenschwanzprotein, ein Autolysin oder ein Bakteriocin.

Die vorliegende Erfindung betrifft ferner ein rekombinant hergestelltes Polypeptid mit der biologischen Aktivität Zellwände von Bakterien zu lysieren, wobei die Aminosäuresequenz des Polypeptids gegenüber der Wildtyp-Sequenz an Aminosäurepositionen Modifikationen aufweist, die von Proteasen erkannt und/oder nach der die Proteasen die Polypeptide spalten.

Überraschenderweise stellte sich heraus, dass die Zellwand lysierenden Enzyme in ihrer Aminosäuresequenz so verändert werden können, dass eine erhöhte Proteasestabilität erreicht werden kann, ohne dass man stabilisierende Substanzen zusetzen muss. Es ist allgemein bekannt, dass verschiedene Proteasen an einem bestimmten Aminosäurerest die Polypeptidkette schneiden oder Aminosäuresequenzmotive für ihre enzymatische Aktivität benötigen. Proteaseschnittstellen in Proteinen können bei vorhandener Aminosäuresequenz mit geeigneten Sequenzanalyseprogrammen vorausgesagt werden (z.B. ExPASy PeptideCutter, Gasteiger et al., Protein Identification and Analysis Tools on the ExPASy Server in John M. Walker (ed): The Proteomics Protocols Handbook, Humana Press (2005)). So ergeben sich für ein Protein mittlerer Größe mit etwa 300 bis 400 Aminosäureresten in der Regel weit mehr als 100 potentielle Proteaseschnittstellen, so dass eine Stabilisierung von Proteinen nur dadurch zu erreichen ist, dass die Schnittstellen einer Vielzahl von Proteasen verändert werden müssten. Das ist jedoch unpraktikabel, da das Protein seine Wirkung und Aktivität verliert, wenn zu viele Aminosäuremodifikationen eingeführt werden. Eine weitere Möglichkeit zur Bestimmung von proteasesensitiven Bereichen neben der Vorhersage anhand der Aminosäuresequenz, vor allem für Proteasen, bei denen keine Erkennungssequenz bekannt ist oder deren Identität man nicht kennt, ist die experimentelle Bestimmung der tatsächlichen Schnittstellen durch einen Verdau der Proteine mit kommerziell erhältlichen Proteasen. Ferner können hierzu aus Organismen oder Proben isolierte Proteasen verwendet werden. Insbesondere können die Proben, die Proteasen enthalten wie z.B. Lebensmittelproben, Umweltproben, Organextrakte, medizinische Proben verwendet werden, ohne die Proteasen vorher zu isolieren. Anschliessend werden die Versuchsansätze einer Analyse der entstehenden Abbauprodukte mit Hilfe von Sequenzbestimmung (z.B. N- oder C-terminale Ansequenzierung, Peptidmapping in Verbindung mit Massenspektrometrie) und Größenbestimmung (z.B. Massenspektrometrie, Bandenanalyse von SDS-Gelen, analytische Gelfiltration) unterzogen. Tatsächlich haben die Erfinder in vielen Zellwand lysierenden Enzymen Schnittstellen und Aminosäuresequenzmotive für Proteasen gefunden. Das erscheint überraschend, wenn man davon ausgeht, dass die Zellwand lysierenden Enzyme eigentlich auf eine hohe Stabilität hin evolviert sein müssten. Überraschenderweise zeigte sich jedoch, dass die Schnittstellen und Aminosäuresequenzmotive für die Proteasen in geringer Anzahl, häufig sogar in singulärer Form in den Zellwand lysierenden Enzymen vorhanden und häufig auch in homologen Proteinen konserviert sind. Dadurch ist eine Modifikation einiger weniger oder sogar nur einer Proteaseschnittstelle ausreichend, um die Stabilität der Zellwand lysierenden Enzyme zu erhöhen.

In der vorliegenden Erfindung werden vorzugsweise nur solche Proteaseschnittstellen verändert, die von bestimmten, potentiell am Wirkort der Zellwand lysierenden Enzyme vorhandenen Proteasen erkannt werden. Dabei werden die Aminosäuresequenzen der Zellwand lysierenden Enzyme so modifiziert, dass sie von den spezifischen Proteasen nicht mehr erkannt und folglich nicht mehr geschnitten werden. Das führt zu einer erhöhten Stabilität der Zellwand lysierenden Enzyme gegenüber am Wirkort vorhandenen Proteasen. Somit wird ein proteasebedingter Verlust der Aktivität der Zellwand lysierenden Enzyme verhindert und eine längere Wirksamkeit erzielt.

Es gibt eine Reihe von Proteasen, die für ihre enzymatische Aktivität eine spezifische Erkennungssequenz in ihren Substratproteinen benötigen. Einige Beispiele dafür sind in der folgenden Tabelle aufgeführt. Dem Fachmann sind eine Vielzahl weiterer Proteasen bekannt wie sie beispielsweise in der Merops Peptidase Datenbank (Rawlings et al., 2008, Nucleic Acids Res 36, D320-D325) beschrieben sind. Das Nachfolgende gilt jedoch auch für Proteaseschnittstellen unbekannter Proteasen, die experimentell bestimmt werden können. P1 bezeichnet die Position der Aminosäure, nach der geschnitten wird, P4, P3 und P2 sind die Positionen N-terminal vor der Schnittstelle P1. Die Bezeichnung P1' und P2' sind die Positionen C-terminal im Anschluss an P1. Das bedeutet, dass die Proteasen die Polypeptidkette zwischen P1 und P1' spalten. Die anstelle der Aminosäurereste aufgeführten Großbuchstaben stellen die international verwendeten Bezeichnungen der Aminosäurereste dar und sind allgemein bekannt. Werden in der Beschreibung der vorliegenden Erfindung die Großbuchstaben verwendet, sind die entsprechenden Aminosäurerest gemeint.

**Tabelle 1**

| Protease | Schnittstelle | | | | | |
|---|---|---|---|---|---|---|
| | P4 | P3 | P2 | **P1** | P1' | P2' |
| Caspase 1 | F, W, Y oder L | - | H, A oder T | **D** | nicht P, E, D, Q, K oder R | - |
| Caspase 2 | D | V | A | **D** | nicht P, E, D, Q, K oder R | - |
| Caspase 3 | D | M | Q | **D** | nicht P, E, D, Q, K oder R | - |
| Caspase 4 | L | E | V | **D** | nicht P, E, D, Q, K oder R | - |
| Caspase 5 | L oder W | E | H | **D** | - | - |
| Caspase 6 | V | E | H oder I | **D** | nicht P, E, D, Q, K oder R | - |
| Caspase 7 | D | E | V | **D** | nicht P, E, D, Q, K oder R | - |
| Caspase 8 | I oder L | E | T | **D** | nicht P, E, D, Q, K oder R | - |
| Caspase 9 | L | E | H | **D** | - | - |
| Caspase 10 | I | E | A | **D** | - | - |
| Clostripain (Clostridiopeptidase B) | - | - | - | **R** | - | |
| Enterokinase | D oder N | D oder N | D oder N | **K** | - | - |
| Faktor Xa | A, F, G, I, L, T, V oder M | D oder E | G | **R** | - | - |
| Plasmin (Fibrinolysin, Fibrinase) | - | - | - | **K or R** | - | - |
| Streptopain (Streptococcus Peptidase A) | - | - | F | **F** | Y | - |
| Bacillolysin (*Bacillus subtilis* neutrale Protease) | - | G oder F oder A | G oder F | **L oder F** | A oder G oder L | - |
| Granzyme B | I | E | P | **D** | - | - |
| Staphylococcus Peptidase I (V8 Protease) | - | - | nicht E | **E** | - | - |
| Thrombin | - | - | G | **R** | G | - |
| | A, F, G, I, L, T, V oder M | A, F, G, I, L, T, V, W or A | P | **R** | nicht D, E | nicht D, E |

Vorzugsweise betrifft die vorliegende Erfindung ein modifiziertes Polypeptid mit der biologischen Aktivität Zellwände von Bakterien zu lysieren, wobei das erfindungsgemäße Polypeptid gegenüber der natürlicherweise vorkommenden eine oder mehrere Modifikationen in seiner Aminosäuresequenz an einer oder mehreren der in Tabelle 1 aufgezeigten Positionen oder in experimentell bestimmten Proteaseschnittstellen aufweist, wobei die eine Modifikation oder die mehreren Modifikationen an den in Tabelle 1 aufgeführten oder experimentell bestimmten Aminosäuren von Proteaseschnittstellen vorkommen. Dadurch erhöht sich die Stabilität des Polypeptids gegenüber der oder den Proteasen, deren Erkennungssequenz entsprechend geändert wurde.

In einer bevorzugten Ausführungsform der vorliegenden Erfindung wird der Aminosäurerest an der Position, nach der geschnitten wird (P1) durch einen anderen geeigneten Aminosäurerest ersetzt, so dass das erfindungsgemäße Polypeptid nicht mehr von der Protease geschnitten wird, dessen Erkennungsstelle entsprechend modifiziert wurde. Statt eines Austausches des Aminosäurerestes an Position P1 oder auch zusätzlich zu einem Austausch an Position P1 können weitere Aminosäurereste in der Erkennungsstelle vorzugsweise die in Tabelle 1 aufgeführten Positonen an P4, P3, P2, P1' und/oder P2', durch andere Aminosäurereste ausgetauscht werden. Vorzugsweise werden pro Erkennungsstelle 6, 5, 4, 3, 2, 1 Sustitutionen vorgenommen, besonders bevorzugt insbesondere 1 bis 3 Substitutionen.

In einer weiteren Ausführungsform der vorliegenden Erfindung können in einem Polypeptid eine oder mehrere Proteaseschnittstellen wie zuvor beschrieben modifiziert werden.

Wichtig ist, dass die erfindungsgemäße Proteinvariante nach der Mutation nicht nur eine höhere Stabilität gegenüber einem Proteaseabbau aufweist, sondern auch weiterhin ihre Zellwand lysierende Aktivität behält. Die Aminosäuresubstitution wird daher wie nachfolgend beschrieben durchgeführt.

Im Stand der Technik sind eine Reihe von essentiellen Aminosäureresten bekannt, die für die Aktivität der Zellwand lysierenden Enzyme bekannt sind z.B. konservierte Cysteine und Histidine bei verschiedenen Amidasen oder CHAP-Domänen. Die entsprechenden essentiellen Reste können auch mit Sequenzanalyseprogrammen gefunden werden, die speziell nach konservierten Domänen suchen (z.B. Conserved Domain Database (CDD) über NCBI (Marchler-Bauer et al., 2005, Nucleic Acids Res. 33, D192-6), Pfam (Finn et al., 2006, Nucleic Acids Research 34, D247-D251) oder SMART (Schultz et al., 1998, Proc. Natl. Acad. Sci. USA 95, 5857-5864, Letunic et al., 2006, Nucleic Acids Res 34, D257-D260).

Um die Stabilität, Struktur und Funktion der erfindungsgemäßen Polypeptide möglichst wenig zu destabilisieren, werden die auszutauschenden Aminosäurereste der Proteaseerkennungssequenz durch Aminosäurereste ersetzt, die von ihrer Struktur und Biochemie her möglichst nah verwandt sind und dennoch von der Protease nicht erkannt werden. Es ist mittlerweile allgemeines Fachwissen die 20 natürlich vorkommenden Aminosäuren in bestimmte Gruppen einzuteilen, die von ihrer Biochemie her unterschiedlich sind (z.B. saure, basische, hydrophobe, polare, aromatische Aminosäuren), innerhalb derer die Aminosäuren jedoch zueinander verwandt sind. Bevorzugte Austausche für die Aminosäurereste in den erfindungsgemäßen Polypeptiden sind in Tabelle 2 zusammengefasst. Die in Spalte 2 genannten Austauschmöglichkeiten stellen konservative Aminosäurealternativen dar, die entweder von ihrer Struktur her und/oder von ihrer Funktion her besonders ähnlich zum Aminosäurerest im Wildtyp sind. Dies sind besonders bevorzugte Austauschvarianten. Möglich sind jedoch für die erfindungsgemäßen Zellwand lysierenden Polypeptide auch weitere Austauschvarianten, die in Spalte 3 genannt sind.

**Tabelle 2**

| Aminosäure im Wildtyp | Konservativer Austausch | Weitere Austauschmöglichkeiten |
|---|---|---|
| A (Ala) | S | T, G, V, E, D |
| N (Asn) | Q, D | K, H, I, T, S, A |
| D (Asp) | E, N | A, G, S |
| R (Arg) | K | S, Q, A |
| C (Cys) | S | M, G, A |
| E (Glu) | D, Q | A, G, S, A |
| Q (Gln) | N, E | H, L, R, A |
| G (Gly) | A | S, N, D |
| H (His) | Q, N | R, A |
| I (Ile) | V, L | T, A |
| L (Leu) | V, I | M,F,A |
| K (Lys) | R | N, T, S, Q, A |
| M (Met) | L | K, V, I, C, A |
| F (Phe) | Y | L, I, M, A |
| P (Pro) | A | S, Q |
| S (Ser) | A, T | G, N, K |
| T (Thr) | S, A | V, K, N |
| W (Trp) | Y | F, S, R, A |
| Y (Tyr) | F | H, N, C, A |
| V (Val) | I, A | L, T |

Eine weitere Möglichkeit zu bestimmen, welcher Aminosäurerest anstelle des zu substituierenden eingebaut wird, stellt die Dayhoff-Matrix dar; siehe z.B. in Creighton (Proteins: Structure and Molecular Properties, 2nd Ed., 1984, Freeman, New York). Aus Untersuchungen homologer Proteine ist bekannt, dass die rein statistisch zufällige Mutation auf Nukleinsäureebene sich nicht in der Aminosäuresequenz der entsprechenden Proteine widerspiegelt. Es scheint somit für bestimmte Aminosäurepositionen in den homologen Proteinen ein Selektionsdruck auf bestimmte Aminosäurereste zu bestehen, was in der Dayhoff-Matrix angegeben ist.

Eine weitere Möglichkeit zu bestimmen, welcher Aminosäurerest anstelle des zu substituierenden eingebaut wird, bedient sich der Hilfe von Sequenzanalyseprogrammen z.B. des Programms BLAST, siehe Altschul et al., 1990, J. Mol. Biol., 215, 403-410. Damit kann nach verwandten Proteinen zu den zu mutierenden Zellwand lysierenden Enzymen gesucht werden. Der Suchalgorithmus findet dabei Ähnlichkeiten auf Sequenzebene. Wird auf Aminosäuresequenzebene gesucht, ist das Programm "Protein BLAST" geeignet, funktionale und/oder evolutionäre Verwandtschaften aufzeigen. Findet man in einem Bereich von etwa 100 oder 50 oder 20 Aminosäureresten als Grenze um die potentielle Proteaseschnittstelle in nah Verwandten Proteinen Aminosäurevarianten mit mindestens etwa 60% oder mindestens etwa 80% oder mindestens etwa 90 % Identität, kann die Aminosäuresequenz, die von der Proteaseerkennungssequenz abweicht, in das erfindungsgemäße Polypeptid eingebaut werden. Die Aminosäuresubstitutionen können dabei von den in Tabelle 2 aufgeführten Austauschmöglichkeiten abweichen.

Es gibt eine Reihe von bekannten Endolysinen, die Proteaseschnittstellen, wie sie in Tabelle 1 dargestellt sind, aufweisen, insbesondere Schnittstellen für die Proteasen Thrombin, Caspase, Clostripain oder V8 Protease. Insbesondere Thrombin- oder Caspase-Schnittstellen sind häufig singulär und können erfindungsgemäß substituiert werden, so dass sich ein stablilisiertes Endolysin ergibt. In einer bevorzugten Ausführungsform wird das erfindungsgemäße Polypeptid in einer oder mehreren Thrombinerkennungssequenzen modifiziert. Dabei wird der mit R bezeichnete Aminosäurerest an Position P1 vorzugsweise gegen den mit K bezeichneten Aminosäurerest ausgetauscht. Ein Austausch gegen S, Q oder A ist ebenfalls bevorzugt. In der ersten Variante der Thrombinerkennungssequenz (G; R; G) können zusätzlich oder alternativ zum Austausch des R die beiden G oder nur eines davon gegen vorzugsweise A ausgetauscht werden. In der zweiten Variante der Thrombinerkennungssequenz (P; R; nicht D, E; nicht D, E) wird ebenfalls vorzugsweise das R gegen ein K oder gegen ein S, Q oder A ausgetauscht. Zusätzlich oder als Alternative kann das P an Position P2 gegen ein A, ein S oder Q ausgetauscht werden. Ferner können zusätzlich oder als Alternative eine oder beide Positionen P1' und P2' mit einem D oder E substituiert werden.

In einer weiteren bevorzugten Ausführungsform wird der Aminosäurerest D an der P1 Position einer Caspaseschnittstelle gegen N oder A ausgetauscht. Der Aminosäurerest R an der P1 Position einer Clostripainschnittstelle wird vorzugsweise gegen K oder A ausgetauscht, der Aminosäurerest E an der P1 Position einer V8 Proteaseschnittstelle gegen Q oder A. Weitere bevorzugte Austausche sind in Tabelle 2 zusammengefasst, stabilisierende Austausche in der Erkennungsstelle außerhalb der P1 Position können ebenfalls durchgeführt werden.

Besonders bevorzugte Ausführungsformen sind aufgeführt in SEQ ID NO:1, 2, 3, 4 und 5.

Die vorliegende Erfindung betrifft ferner ein rekombinant hergestelltes Polypeptid mit der biologischen Aktivität Zellwände von Bakterien zu lysieren, wobei die Aminosäuresequenz des Polypeptids gegenüber der Wildtyp-Sequenz Aminosäureadditionen aufweist. Vorzugsweise weist das erfindungsgemäße Polypeptid ein, zwei, drei oder vier Additionen von Aminosäureresten auf, wobei die Aminosäurereste zusammenhängend oder unabhängig voneinander eingefügt werden können. Bevorzugt ist die Addition von einer oder zwei Aminosäuresten, insbesondere bevorzugt ist die Addition von einem Aminosäurerest, ganz besonders bevorzugt ist die Addition von einem Aminosäurerest an Position zwei. Die Aminosäureadditionen können in Polypeptide eingefügt werden, die an Aminosäurepositionen Modifikationen aufweisen, die von Proteasen erkannt und/oder nach der die Proteasen die Polypeptide spalten, oder auch in Polypeptide, in die derartige Modifikationen nicht eingebaut wurden. Besonders bevorzugte Ausführungsformen sind aufgeführt in SEQ ID NO:12-17, 19 und 22.

In einer weiteren bevorzugten Ausführungsform wird das erfindungsgemäße Polypeptid in einer oder mehreren V8 Protease-Erkennungssequenzen modifiziert. Dabei wird der mit E bezeichnete Aminosäurerest an Position P1 mit einem anderen Aminosäurerest vorzugsweise gegen den mit A oder Q bezeichneten Aminosäurerest ausgetauscht. Eine besonders bevorzugte Ausführungsform ist aufgeführt in SEQ ID NO:6.

In einer weiteren bevorzugten Ausführungsform wird das erfindungsgemäße Polypeptid an einer oder mehreren Clostripainschnittstellen modifiziert. Dabei wird der mit R bezeichnete Aminosäurerest an Position P1 mit einem anderen Aminosäurerest vorzugsweise gegen den mit K oder A bezeichneten Aminosäurerest ausgetauscht. Besonders bevorzugte Ausführungsformen sind in SEQ ID NO:7 und 8 aufgeführt.

In einer weiteren bevorzugten Ausführungsform wird das erfindungsgemäße Polypeptid in einer oder mehreren Caspase Erkennungssequenzen modifiziert. Dabei wird der mit D bezeichnete Aminosäurerest an Position P1 mit einem anderen Aminosäurerest vorzugsweise gegen den mit E oder A bezeichneten Aminosäurerest ausgetauscht. Besonders bevorzugte Ausführungsformen sind in SEQ ID NO:9 und 10 aufgeführt.

In einer weiteren bevorzugten Ausführungsform wird das erfindungsgemäße Polypeptid in einer oder mehreren V8 Protease-Erkennungssequenzen und einer oder mehreren ThrombinErkennungssequenzen modifiziert. Dabei wird der mit E bezeichnete Aminosäurerest an Position P1 mit einem anderen Aminosäurerest vorzugsweise gegen den mit Q oder A bezeichneten Aminosäurerest ausgetauscht und der mit R bezeichnete Aminosäurerest an Position P1 mit einem anderen Aminosäurerest vorzugsweise gegen den mit K oder A bezeichneten Aminosäurerest ausgetauscht. Eine besonders bevorzugte Ausführungsform ist in SEQ ID NO:11 aufgeführt.

In einer weiteren bevorzugten Ausführungsform wird das erfindungsgemäße Polypeptid in einer oder mehreren Protease-Erkennungssequenzen, die nach einem Verdau mit Nierenextrakt identifiziert wurden modifiziert. Dabei wird der mit Q bezeichnete Aminosäurerest an Position P1 mit einem anderen Aminosäurerest vorzugsweise gegen den mit N oder A bezeichneten Aminosäurerest ausgetauscht und der mit S bezeichnete Aminosäurerest an Position P1 mit einem anderen Aminosäurerest vorzugsweise gegen den mit T oder A bezeichneten Aminosäurerest ausgetauscht. Besonders bevorzugte Ausführungsformen sind in SEQ ID NO:12 und 13 aufgeführt.

Die im Rahmen der vorliegenden Erfindung eingeführten Aminosäuresubstitutionen können mit Hilfe von molekularbiologischen Standardtechniken erfolgen, die dem Fachmann bekannt sind und z.B. in Sambrook et al., Molecular cloning. A laboratory manual; 2nd ed. Cold Spring Harbor Laboratory Press 1989, aufgeführt sind. So können mit Hilfe von DNA-Primem in die Nukleotidsequenz, die die erfindungsgemäßen Polypeptide codieren, die gewünschten Mutationen eingebaut werden. Die so erhaltenen modifizierten Nukleotidsequenzen können dann in einem geeigneten Wirt, vorzugsweise E.coli, exprimiert und die Polypeptide aufgereinigt werden. Die Nukleotidsequenz codierend die erfindungsgemäßen Polypeptide kann ferner mit dem sogenannten Codon-Usage an die entsprechende Wirtszelle angepasst werden, um eine bessere Expression zu erzielen. Die vorliegende Erfindung betrifft somit die Nukleotidsequenz codierend die erfindungsgemäßen Polypeptide, entsprechende Expressionsvektoren und Wirtszellen zur Herstellung der erfindungsgemäßen Polypeptide.

Die erfindungsgemäßen Polypeptide können mit verschiedenen Tests auf Proteaseempfindlichkeit und/oder Enzymaktivität untersucht werden. Beispiele für solche Test sind Proteaseverdau und anschließende Auftrennung der Fragmente über SDS-Gelelektrophorese, Massenspektrometrie, Zelllysetest auf Agarplatten, Flüssiglysetest durch Messung der Lichtstreuung im Photometer, Zymogrammassay zum Aktivitätstest auf Gelen.

Die erfindungsgemäßen Polypeptide können im medizinischen, therapeutischen, diagnostischen, umwelttechnischen, kosmetischen oder Lebensmittelbereich eingesetzt werden.

Die vorliegende Erfindung betrifft ferner ein Nukleinsäuremolekül, umfassend eine Nukleotidsequenz, die ein erfindungsgemäßes Polypeptid kodiert. Ferner betrifft die vorliegende Erfindung einen Vektor, umfassend ein erfindungsgemäßes Nukleinsäuremoleküle.

Ferner betrifft die vorliegende Erfindung geeignete Wirtszellen zur Expression der erfindungsgemäßen Polypeptide. Vorzugsweise umfasst eine geeignete Wirtszelle zur Expression der erfindungsgemäßen Polypeptide ein erfindungsgemäßes Nukleinsäuremolekül oder einen erfindungsgemäßen Vektor. Vorzugsweise ist eine geeignete Wirtszelle zur Expression der erfindungsgemäßen Polypeptide mit einem erfindungsgemäßen Nukleinsäuremolekül transformiert.

Bei der Modifikation der Erkennungsstellen wird dabei vorzugsweise die Erkennungsstelle solcher Proteasen modifiziert, die in dem gewünschten Anwendungsgebiet erfahrungsgemäß vorkommen. So ist bekannt, dass im Menschen oder im Tier z.B. die Caspasen, Thrombin, Granzyme B, Enterokinase, Faktor Xa vorkommen. Dem Fachmann sind auch gewebsspezifische Proteasen bekannt, z.B. die Proteasen Meprin und Renin, die in der Niere vorkommen. Ferner können darüber hinaus Proteasen aus pathogenen Bakterien vorliegen, die eine Infektion im Mensch oder Tier auslösen oder als Begleitflora mit vorhanden sind. Dazu zählen z.B. die V8 Protease aus *Staphylococcus* oder Clostridiopeptidase B aus Clostridien. Sollen die erfindungsgemäßen Polypeptide in der Therapie solcher Infektionen verwendet werden, werden vorzugsweise die Erkennungsstellen für die Proteasen modifiziert, die in den Bakterien vorliegen, die für eine zu therapierende Infektion verantwortlich sind. Vorzugsweise werden die proteasesensitiven Bereiche der Zellwand lysierenden Enzyme experimentell bestimmt durch einen Verdau der Proteine mit kommerziell erhältlichen Proteasen, aus Organismen oder Proben isolierten Proteasen oder Proben, die Proteasen enthalten wie z.B. Lebensmittelproben, Umweltproben, Organextrakte, medizinische Proben und einer anschliessenden Analyse der entstehenden Abbauprodukte mit Hilfe von Sequenzbestimmung (z.B. N- oder C-terminale Ansequenzierung, Peptidmapping in Verbindung mit Massenspektrometrie) und Größenbestimmung (z.B. Massenspektrometrie, Bandenanalyse von SDS-Gelen, analytische Gelfiltration). Ein Anwendungsgebiet für Zellwand lysierende Enzyme ist die topische Anwendung in Wunden in Form von Salben, Cremes, Tinkturen oder Wundabdeckungen wie Verbänden oder Bandagen, z.B. bei einer Infektion durch Staphylokokken oder Clostridien. Zusätzliche Stabilitätsprobleme für die Zellwand lysierenden Enzyme können dadurch auftreten, dass in der Wundversorgung teilweise Medikamente oder medizinische Produkte verwendet werden, die ebenfalls Proteasen enthalten. Beispielsweise wird bei Wunden der Haut versucht, eine sanfte Wundreinigung durch Fibrinolyse zu erreichen. Beispiele für Proteasen, die in der Wundversorgung eingesetzt werden sind Fibrinolysin, Plasmin, Streptokinase, Clostridiopeptidase A und *Bacillus subtilis* Protease. Vorzugsweise weisen die erfindungsgemäßen Polypeptide ebenfalls Modifikationen der Erkennungsstellen für diese Proteasen auf.

In der Lebensmittel-, Umwelt- und Kosmetikindustrie können ebenfalls eine Vielzahl von Proteasen vorliegen, die entweder von Bakterien produziert werden, die z.B. in der Lebensmittelherstellung verwendet werden wie Laktobazillen, Lactococcen oder verschiedene Bazillenstämme, oder die von unerwünschten Keimen, die lysiert werden sollen, ausgeschieden werden (z.B. *B. cereus, B. subtilis, Cl. perfringens, Cl. botulinum*). In Bakterien, die in der Lebensmittelherstellung verwendet werden, wie z.B. *Lactobacillus acidophilus, L. casei, L. delbrueckii, L. brevis, L. helveticus* oder *Lactococcus lactis* sind beispielsweise jeweils zwischen 60 und 130 Proteasen bekannt. Zusätzlich sind auch in den Lebensmitteln selber Proteasen vorhanden. Vorzugsweise weisen die erfindungsgemäßen Polypeptide solche Modifikationen der Erkennungsstellen von Proteasen auf, die in der Lebensmittel-, Umwelt- und Kosmetikindustrie vorkommen.

Die vorliegende Erfindung betrifft ferner das erfindungsgemäße Polypeptid als Arzneimittel oder pharmazeutische Zusammensetzung sowie die Verwendung des erfindungsgemäßen Polypeptids als Arzneimittel oder pharmazeutische Zusammensetzung zur Prävention oder Therapie von Erkrankungen, die durch gram positive Bakterien insbesondere Clostridien, Bazillen, Listerien, Staphylokokken, Lactobazillen, Enterokokken, Aerokokken, Pediokokken, Streptokokken, Mycoplasmen und/oder Leuconostoc hervorgerufen werden.

Die vorliegende Erfindung betrifft ferner das erfindungsgemäße Polypeptid als Diagnostikum oder diagnotische Zusammensetzung sowie die Verwendung des erfindungsgemäßen Polypeptids als Diagnostikum oder diagnotische Zusammensetzung in der Medizin zum Nachweis von Erkrankungen, insbesondere die durch gram positive Bakterien insbesondere Clostridien, Bazillen, Listerien, Staphylokokken, Lactobazillen, Enterokokken, Aerokokken, Pediokokken, Streptokokken, Mycoplasmen und/oder Leuconostoc hervorgerufen werden.

Das erfindungsgemäße Polypeptid wird in der Diagnostik dazu verwendet, um die nachzuweisenden Bakterien spezifisch zu lysieren, so dass sich ein oder mehrere Detektionsschritte anschließen können, die spezifische Zellbestandteile der Bakterien nachweisen wie z.B. DNA, RNA, Enzyme, Zellwandkomponenten. Die dafür geeigneten Verfahren z.B. PCR, NASBA, Hybridisierung, Antikörper basierte Nachweise wie ELISA, biochemischer Nachweise für bestimmte spezifische Enzyme, colorimetrische Nachweise sind aus dem Stand der Technik bekannt.

Darüber hinaus betrifft die vorliegende Erfindung die Verwendung des erfindungsgemäßen Polypeptids zur Unterdrückung des Wachstums oder des Nachweises von gram positiven Bakterien in der Umwelt-, Lebensmittel- oder Kosmetikindustrie.

Die folgenden Ausführungsbeispiele dienen der Erläuterung der Erfindung, sind jedoch nicht als einschränkend aufzufassen.

### Beispiel 1: Proteaseverdau von PlyPitti26

Es wurde ein Verdau von PlyPitti26 mit den Proteasen Thrombin und V8 durchgeführt. Jeweils 90 µl Proteinlösung (Proteinkonzentration 1,35 mg/ml) wurden mit 10 µl Thrombin und in Kontrollansätzen nur mit Plasmin versetzt. Für den V8 Protease Verdau wurden 99 µl Proteinlösung und 1 µl Proteaselösung verwendet. Die Konzentration der Proteasestammlösungen betrug jeweils 500 µg/ml. Die Probenansätze wurden über Nacht bei 25 °C in einem 20 mM Tris/HCl pH 7.5, 10 mM DTE, 0.1 mM ZnSO₄ Puffer inkubiert. Ein Teil der Proben wurde mit SDS-Gel-Auftragspuffer versetzt und nach Aufkochen auf SDS-Polyacrylamidgelen analysiert.

Es wurde festgestellt, dass PlyPitti26 durch Thrombin vollständig abgebaut wird, wobei zwei relativ große Proteinfragmente entstanden, von denen das eine die CHAP-Domäne enthielt und das andere die Amidase-Domäne und die CBD. Bei einem Verdau mit V8 Protease entstanden mehrere kleinere, aber ebenfalls definierte Proteinfragmente. Bei einem Kontrollverdau mit Plasmin erfolgte unter den gegebenen Bedingungen keine Fragmentierung des Proteins. Die Ergebnisse sind in Fig. 2A gezeigt.

### Beispiel 2: Aktivitätstest von unbehandeltem und mit Proteasen behandeltem PlyPitti26

In weiteren Versuchen wurden die Proben im Flüssiglysetest auf die Lyseaktivität gegenüber *Staphylococcus* Zellen getestet. Der Flüssiglysetest ist ein Aktivitätstest für Zellwand lysierende Enzyme, bei dem die bakterielle Zelllyse in Echtzeit über Lichtstreuung im Photometer gemessen wird. Die Absorption bei einer Wellenlänge von 600 nm ist dabei ein Maß für die Anzahl an vorhandenen Bakterienzellen. Lysieren die Bakterienzellen wird die Probenlösung nach und nach klar und die gemessene Absorption nimmt im Idealfall bis auf einen Wert von Null ab. *Staphylococcus* Bakterien wurden in BHI-Medium bis zu einer OD₆₀₀ von ungefähr 0,8 kultiviert. Die nicht hitzeinaktivierten Zellen wurden geerntet und in TBST Puffer (20 mM Tris/HCl pH 7.5, 60 mM NaCl, 0.1 % Tween, 2 mM CaCl₂) zu einer OD₆₀₀ von ungefähr 1 resuspendiert. 990 µl Zellsuspension wurde jeweils mit 10 µl Enzymlösung versetzt (Enzymkonzentration im Test 10 µg/ml) und bei 30 °C die Abnahme der Absorption bei 600 nm verfolgt.

Es wurde festgestellt, dass bei nicht verdautem PlyPitti26 und bei der Plasminkontrolle nach etwa 1000 s eine vollständige Lyse der Staphylokokken eingetreten ist, während bei den mit Thrombin und V8 Protease verdauten Proben keine sichtbare Zelllyse mehr stattgefunden hat, das Zellwand lysierende Enzym also vollständig inaktiviert wurde. Die Ergebnisse sind in Fig. 2B gezeigt.

### Beispiel 3. Stabilisierung von CHAP-AmiPitti26-CBDUSA

Eine gegen Staphylokokken wirksame synthetisch hergestellte Endolysinvariante, die aus den enzymatisch aktiven Domänen CHAP und Ami des Endolysins von PlyPitti26 besteht und aus der Zellwand bindenden Domäne (CBD) des Endolysins des Prophagen ΦSA2USA, der aus dem Genom des MRSA Stamms USA300 stammt (Diep et al., The Lancet, 2006, 367, 731-739; Datenbank Zugangsnummer NC_007793) wird als CHAP-AmiPitti26-CBDUSA bezeichnet.

Um dieses Protein gegen Thrombinverdau und Verdau durch V8 Protease zu stabilisieren wurden mittels zielgerichteter Mutagenese sowohl die Thrombin-Schnittstelle als auch die im Linker zwischen CHAP- und Amidase-Domäne liegenden V8-Schnittstellen durch Aminosäuresubstitutionen modifiziert. Die zu diesem Zweck durchgeführten erfindungsgemäßen Einzel-, Doppel-, Dreifach und Vierfachmutationen sind in Tabelle 3 zusammengefasst. Für alle in Tabelle 3 aufgeführten Mutanten wurde nach der Mutation noch Endolysinaktivität gegen Staphylokokken im Plattenlysetest wie nachstehend beschrieben nachgewiesen. Die in der Tabelle aufgeführten Aminosäurepositionen beziehen sich auf die jeweiligen Positionen der Wildtypsequenz bzw. der Positionen in modifizierten Polypeptiden, in denen keine Aminosäurereste zugefügt wurden. Beispielsweise ist die Position E163 in einer Ausführungsform eines erfindungsgemäßen Polypeptids mit einer Aminosäureaddition an Position zwei E164, die Position E167 ist dann E168. Bei mehr als einer Aminosäureaddition verschiebt sich die Position entsprechend. Wird ein Aminosäurerest erst an Position 174 eingefügt, so bleibt die Position E163 gleich, aber die Position E189 verändert sich zu E 190.

**Tabelle 3**

| Mutante | Schnittstelle | Austausch |
|---|---|---|
| | V8: V8 Protease | |
| | T: Thrombin | |
| 1 | E163 (V8) | 163Q |
| | R167 (T) | 167K |
| 2 | E163 (V8) | 163Q |
| | R167 (T) | 167A |
| 3 | E163 (V8) | 163A |
| | R167 (T) | 167K |
| 4 | E163 (V8) | 163A |
| | R167 (T) | 167A |
| 5 | E179 (V8) | 179Q |
| 6 | E179 (V8) | 179A |
| 7 | E189 (V8) | 189Q |
| 8 | E189 (V8) | 179A |
| 9 | E163 (V8) | 163Q |
| | R167 (T) | 167A |
| | E179 (V8) | 179Q |
| | E189 (V8) | 189Q |
| 10 | E163 (V8) | 163Q |
| | R167 (T) | 167A |
| | E179 (V8) | 179A |
| | E189 (V8) | 189Q |
| 11 | E163 (V8) | 163A |
| | R167 (T) | 167A |
| | E179 (V8) | 179Q |
| | E189 (V8) | 189Q |
| 12 | E163 (V8) | 163A |
| | R167 (T) | 167A |
| | E179 (V8) | 179A |
| | E189 (V8) | 189Q |
| 13 | R167 (T) | 167A |
| 14 | E163 (V8) | 163A |
| 15 | E163 (V8) | 163Q |
| 16 | R167 (T) | 167K |
| 17 | E179 (V8) | 179A |
| | E189 (V8) | 189Q |
| 18 | E179 (V8) | 179Q |
| | E189 (V8) | 189Q |
| 19 | E163 (V8) | 163Q |
| | R167 (T) | 167A |
| | E189 (V8) | 189Q |
| 20 | E163 (V8) | 163A |
| | R167 (T) | 167A |
| | E189 (V8) | 189Q |

Die Endolysine wurden in einer Konzentration von 1mg/ml in den Verdau eingesetzt. Für den Thrombinverdau wurden 90µl Protein und 10µl humanes Thrombin (Stammlösung mit 50µg/ml) eingesetzt. Die Probenansätze wurden über Nacht bei 25 °C in Puffer (20 mM Tris/HCl pH 7.5, 10 mM DTE, 0.1 mM ZnSO₄) inkubiert. Ein Teil der Proben wurde mit SDS-Gel-Auftragspuffer versetzt und nach Aufkochen auf SDS-Polyacrylamidgelen analysiert. Die Mutation von R an Position 167 gegen A führt zu einer vollständigen Insensibilisierung gegen Thrombin. Gleiches galt für die Mutation von R an Position 168 gegen A, nach Addition von A an Aminosäureposition zwei (CHAP-Ami_Pitti26-CBD-USA-Add2).

Nicht modifiziertes CHAP-Ami_Pitti26-CBD-USA, CHAP-Ami_Pitti26-CBD-USA-Add2 und die jeweilige Mutante 4 (E163A, R167A, bzw. E164A, R1678) wurden ebenfalls mit V8 Protease verdaut. Die Endolysine wurden in einer Konzentration von 1mg/ml in den Verdau eingesetzt. Für den V8 Protease Verdau wurden 99 µl Protein und 1 µl V8 Protease (Stammlösung mit 500 µg/ml) eingesetzt. Die Probenansätze wurden für 15 min bzw. 60 min in 20 mM Tris/HCl pH 7.5, 10 mM DTE, 0.1 mM ZnSO₄ Puffer inkubiert. Ein Teil der Proben wurde mit SDS-Gel-Auftragspuffer versetzt und nach Aufkochen auf SDS-Polyacrylamidgelen analysiert. Die Mutation von E an Position 163, bzw. 164 gegen A führt zwar nicht zu einer vollständigen Insensibilisierung gegenüber V8 Protease, jedoch ist die erfindungsgemäße Mutante gegenüber dem Wildtyp, bei dem ein vollständiger Verdau eintritt, deutlich stabilisiert. Die modifizierte Thrombinschnittstelle an Position 167/168 spielte bei dem durchgeführten V8 Protease-Verdau keine Rolle. Bei den modifizierten Polypeptiden sind sowohl nach 15 min als auch nach 60 min noch zwei Fragmente von etwa 40 kDa vorhanden, die bei den nicht modifizeirten Polypeptiden nach 15 min nur noch schwach und nach 60 min gar nicht mehr vorhanden sind.

### Beispiel 4. Lysetest mit Proteasesensitiven und Proteaseresistenten Endolysinen

Um zu untersuchen, ob die erfindungsgemäßen Polypeptide nach den Aminosäureaustauschen noch die gewünschte Aktivität besitzen, wurden sie in einem Plattenlysetest mit verschiedenen *Staphylococcus* Stämmen getestet. Die Bakterienstämme wurden über Nacht in 5 ml Kulturen in BHI-Medium angezogen und die Bakterienzellen 5 min bei 4000 UpM in der Tischzentrifuge zentrifugiert. Das Zellpellet wurde anschließend in 200 µl PBS-Puffer (10 mM NaPhosphat, 144 mM NaCl, 50 mM KCl, pH 7,4) resuspendiert und die Zellen 30 min bei 80 °C hitzeinaktiviert. Die hitzeinaktivierten Zellen wurden mit 15 ml Topagar in Petrischalen gegossen und getrocknet. Anschließend wurden jeweils 5 µg Protein aufgetüpfelt und die Agarplatten für 1 h bei 30 °C inkubiert. Enstehende Zelllysehöfe an den Stellen der aufgetüpfelten Proteine wurden visuell ausgewertet und je nach Größe mit +++, ++ und + bewertet oder mit -, falls keine Lyse auftrat.

Das Testergebnis für einige erfindungsgemäße Polypeptide ist in Tabelle 4 zusammengefaßt. Tabelle 4

| Staphylococcus Spezies | PlyPitti26 (nicht modifiziert) | Mutante 10 aus Tabelle 3 (E163Q, R 167A, E179A, E189Q) | Mutante 4 aus Tabelle 3 (E163A, R167A) | CHAP-AmiPitti26-CBDUSA (nicht modifiziert) |
|---|---|---|---|---|
| *Staphylococcus aureus* MRSA | +++ | +++ | +++ | +++ |
| *Staphylococcus aureus* MRSA | +++ | +++ | +++ | +++ |
| *Staphylococcus aureus* MRSA | ++ | +++ | +++ | +++ |
| *Staphylococcus epidermis* | +++ | +++ | +++ | +++ |
| *Staphylococcus equorum* | + | ++ | ++ | ++ |
| *Staphylococcus sciuri* | - | ++ | + | ++ |
| *Staphylococcus saprophyticus* | - | +++ | ++ | +++ |
| *Staphylococcus sciuri* | - | - | - | - |
| *Staphylococcus aureus* MRSA | +++ | +++ | +++ | +++ |
| *Staphylococcus epidermidis* | +++ | +++ | +++ | +++ |
| *Staphylococcus epidermidis koagulase-negativ* | ++ | ++ | ++ | ++ |
| *Staphylococcus haemolyticus koapulase-nepativ* | ++ | ++ | ++ | ++ |
| *Staphylococcus epidermidis koagulase-negativ* | +++ | +++ | +++ | +++ |

Sowohl die nicht modifizierten PlyPitti26 als auch CHAP-AmiPitti26-CBDUSA wiesen ein breites Lysespektrum gegenüber verschiedenen *Staphylococcus* Stämmen auf, wobei das CHAP-AmiPitti26-CBDUSA eine etwas bessere Lyseaktivität zeigte. Die beiden in der Tabelle gezeigten erfindungsgemäßen modifizierten Polypeptide Mutante 4 und Mutante 10 mit den Aminosäureaustauschen an der Thrombin- bzw. verschiedenen V8 Proteaseschnittstellen wiesen eine praktisch identische Aktivität wie das nicht modifizierte Protein CHAP-AmiPitti26-CBDUSA auf. Dies zeigte, dass die erfindungsgemäßen Mutationen die Aktivität der Zellwand lysierenden Enzyme nicht negativ beeinflussten. Gleiches galt für nicht modifiziertes CHAP-Ami_Pitti26-CBD-USA-Add2 sowie die entsprechenden modifizierten CHAP-Ami_Pitti26-CBD-USA-Add2 Polypeptide.

### Beispiel 5: Modifizierte Zellwand bindende Enzyme gegen Clostridium difficile

Zwei Endolysine aus verschiedenen *Cl. difficile* Phagen (Zugangsnummern YP_529586 für das Endolysin aus dem Phagen Φ CD119 und YP_001087453 für das Endolysin aus dem Stamm *Cl. difficile* 630) wiesen eine singuläre Caspase 1 Schnittstelle nach Aminosäure D214 auf, wohingegen eine singuläre Thrombinschnittstelle nur bei dem Endolysin aus dem Phagen Φ CD119 an Position 87 auftrat. Aufgrund der hohen Sequenzähnlichkeit der beiden Endolysine, wurde neben den Modifikationen der Caspase 1 Schnittstellen von D214 zu E214 beim Φ CD119 Endolysin R an Position 87 durch K ausgetauscht, das in dem homologen Endolysin aus dem Stamm *Cl. difficile* 630 an dieser Position vorhanden war und von Thrombin nicht als Schnittstelle erkannt wird. Weitere erfindungsgemäße Modifikationen sind in Tabelle 2 dargestellt.

### Beispiel 6: Bestimmung von proteasesensiblen Bereichen innerhalb der Ply511 Sequenz.

Ply511 besitzt aufgrund seiner Aminosäuresequenz 6 potentielle Schnittstellen für Staphylococcus Peptidase I (6 Glutamate). Um herauszufinden, welche Bereiche des Endolysins Ply511 besonders proteaseempfindlich sind, wurden Proteaseverdauexperimente mit Staphylococcus Peptidase I durchgeführt. Für den Staphylococcus Peptidase I Abbau wurden 99 µl Proteinlösung (Konzentration ca. 1 mg/ml) und 1 µl Proteaselösung (Konzentration ca. 0,5 mg/ml) verwendet. Die Probenansätze wurden für verschiedene Zeitintervalle (Minuten bis mehrere Stunden) bei 37 °C in einem Puffer mit 20 mM Tris/HCl, 100 mM NaCl, pH 8,0 inkubiert. Die Proteinbanden wurden auf SDS-Gelen aufgetrennt. Die entstandenen Abbaufragmente wurden auf PVDF (Polyvinylidenfluorid) Membranen geblottet, gut separierbare Banden ausgeschnitten und N-terminal ansequenziert. Es ergaben sich drei bevorzugte Schnittstellen für Staphylococcus Peptidase I nach den Aminosäuren E7, E40 und E89.

### Beispiel 7: Entfernung von Staphylococcus Peptidase I Schnittstellen in Ply511

An den Positionen E7, E40 und E89 wurden Substitutionen entweder als Einzelsubstitution oder in Kombination durchgeführt, so dass in den entstehenden Mutanten andere Aminosäuren außer Glutamat an die jeweiligen Positionen traten, die nicht mehr von Staphylococcus Peptidase I geschnitten werden. Die so erhaltenen Mutanten wurden in den in Beispiel 6 beschriebenen Proteaseverdau eingesetzt und anschließend auf SDS-Gelen analysiert, ob die entsprechenden Abbaubanden noch auftraten oder nicht. Als besonders geeignet erwiesen sich die Mutationen E7A und E7Q sowie E40A und E40Q.

### Beispiel 8: Resistenz gegenüber Thrombin und V8 Protease verschiedener Endolysinvarianten von Plypitti26 und CHAP-AmiPitti26-CBDUSA.

Verschiedene erfindungsgemäße Mutantenkombinationen aus Tabelle 3 wurden in die Plypitti26 Variante CHAP-AmiPitti26-CBDUSA und CHAP-AmiPitti26-CBDUSA-Add2 eingeführt und zum Teil mit weiteren Mutationen kombiniert. Anschließend wurden diese Endolysinvarianten auf ihre Resistenz gegenüber Thrombin und V8 Protease sowie die Enzymaktivität getestet (Nachweis wie in Beispiel 3 beschrieben). Die Ergebnisse sind in Tabelle 5 für CHAP-AmiPitti26-CBDUSA und dessen Modifikationen zusammengefasst. Die Ergebnisse für CHAP-AmiPitti26-CBDUSA-Add2 und dessen Modifikationen (Aminosäurepositionen sind gegenüber den Positionen in der Tabelle jeweils um eine Position weiter Richtung C-Terminus) sind identisch und in Tabelle 5 nicht nochmals wiedergegeben..

**Tabelle 5: Resistenz gegenüber Thrombin und V8 Protease verschiedener Mutanten von CHAP-Amipitti26_CBDUSA(in der Tabelle EADpitti26_CBDUSA bezeichnet) im Vergleich zu Plypitti26-Wildtyp**

| **Endolysin-Konstrukt** | | plypitti26 | EADpitti 26_CBDUSA | EADpitti 26_CBDUSA | EADpitti26 _CBDUSA | EADpitti26 _CBDUSA | EADpitti26 _CBDUSA | EADpitti26 _CBDUSA | EADpitti26 _CBDUSA |
|---|---|---|---|---|---|---|---|---|---|
| **Mutationen** | | | E163A, R167A, Y200H | E163A, R167A, E179A, E189Q, Y200H | L55H, L56T, E163A, R167A, Y200H | L55H, L56T, E163A, R167A, E179A, E189Q, Y200H | L55H, L56T, | E163A, R167A | |

| **Eigenschaft** | **Assay** | | | | | | | | |
|---|---|---|---|---|---|---|---|---|---|
| Thrombin-resistenz | SDS-PAGE | - | +++ | ++ | +++ | +++ | - | +++ | + |
| Thrombinresistenz | Flüssig lyse | - | - | - | ++ | ++ | - | - | + |
| V8 Proteaseresistenz | SDS-PAGE | - | - | + | - | + | - | - | - |

Es zeigte sich, dass die erfindungsgemäßen Endolysinvarianten CHAP-Amipitti26_CBDUSA(E163A, R167A, Y200H), CHAP-Amipitti26_CBDUSA(E163A, R167A, E179A, E189Q, Y200H), CHAP-Amipitti26_CBDUSA(L55H, L56T, E163A, R167A, Y200H), CHAP-Amipitti26_CBDUSA(L55H, L56T, E163A, R167A, E179A, E189Q, Y200H) und CHAP-Amipitti26_CBDUSA(E163A, R167A) sowie CHAP-Amipitti26_CBDUSA-Add2 (E164A, R168A, Y201H), CHAP-Amipitti26_CBDUSA-Add2 (E164A, R168A, E180A, E190Q, Y201H), CHAP-Amipitti26_CBDUSA-Add2 (L56H, L57T, E164A, R168A, Y201H), CHAP-Amipitti26_CBDUSA-Add2 (L56H, L57T, E164A, R168A, E180A, E190Q, Y201H) und CHAP-Amipitti26_CBDUSA-Add2 (E164A, R168A) eine gute bis sehr gute Resistenz gegenüber Thrombinverdau aufwiesen. Die Endolysinvarianten CHAP-Amipitti26_CBDUSA(L55H, L56T, E163A, R167A, Y200H) und CHAP-Amipitti26_CBDUSA(L55H, L56T, E163A, R167A, E179A, E189Q, Y200H) sowie CHAP-Amipitti26_CBDUSA-Add2 (L56H, L57T, E164A, R168A, Y201H) und CHAP-Amipitti26_CBDUSA-Add2 (L56H, L57T, E164A, R168A, E180A, E190Q, Y201H) zeigten auch im Flüssiglysetest eine hohe Enzymaktivität nach Thrombinverdau. Die Endolysinvarianten CHAP-Amipitti26_CBDUSA(E163A,R167A, E179A, E189Q, Y200H) und CHAP-Amipitti26_CBDUSA(L55H, L56T, E163A, R167A, E179A, E189Q, Y200H) sowie CHAP-Amipitti26_CBDUSA-Add2 (E164A, R168A, E180A, E190Q, Y201H) und CHAP-Amipitti26_CBDUSA-Add2 (L56H, L57T, E164A, R168A, E180A, E190Q, Y201H) wiesen eine verbesserte Resistenz gegenüber V8 Proteaseverdau auf. Besonders die Substitutionen E179A und E189Q bzw. E180A und E190Q erwiesen sich hier als vorteilhaft.

### Beispiel 9: Endolysine mit erfindungsgemäßen Proteaseschnittstellen

Ob sich eine erfindungsgemäße Substitution eines Aminosäurerestes als vorteilhaft erweist, lässt sich anhand der weiter oben beschriebenen Nachweise für Proteasestabilität und Endolysinaktivität nachweisen. Eine Reihe von Beispielen für Endolysine, die Vertreter aus verschiedenen Gattungen von Bakterien lysieren und die sich erfindungsgemäß gegen Proteaseabbau stabilisieren lassen, sind in Tabelle 6 aufgezeigt. Die Proteaseschnittstellen wurden mit dem Programm PeptideCutter (Gasteiger et al., Protein Identification and Analysis Tools on the ExPASy Server in John M. Walker (ed): The Proteomics Protocols Handbook, Humana Press (2005)) bestimmt.

Tabelle 6: Endolysine mit Proteaseschnittstellen nach Tabelle 1

| | | **Proteaseschnittstellen für** | | | |
|---|---|---|---|---|---|
| **Endolysin (Lyse von Bakterien der Gattung)** | **Quelle für die Aminosäures equenz (NCBI Zugangsnummer)** | **Thrombin; Nummer von Aminosäure P1 und Erkennungssequenz** | **Caspase1; Nummer von Aminosäure P1 und Erkennungssequenz** | **Clostripain; Anzahl** | **V8 Protease; Anzahl** |
| PlyPitti26 (Staphylococcus) | Neu | 167 (TAP**R**) | | 20 | 24 |
| CHAP-AmiPitti26-CBDUSA (Staphylococcus) | Neu | 167 (TAP**R**) | | 21 | 23 |
| Amidase of Staphylococcus phage SAP-2 | (YP_0014915 39) | 39) | 42 (YITDG) | 7 | 9 |
| E1 Endolysin (Enterococcus) | (NP_814147) | 106 (FLP**R**) | 358 (YGT**D**Y) | 6 | 15 |
| E2 Endolysin (Enterococcus) | (NP_815016) | | 330 (YIA**D**G) und Caspase 10 31 (IEA**D**) | 13 | 13 |
| E5 Endolysin (Enterococcus) | (NP_815749) | | 147 (WLA**D**Y) | 8 | 13 |
| Fab25 (Enterococus) | Neu | | 112 (LLH**D**L) | 14 | 9 |
| B30-PlyGBS (Streptococcus) | (AAR99416) | | 388 (LST**D**Y) | 12 | 18 |
| Pal amidase (Streptococcus) | (CAB07986) | | | 14 | 13 |
| Cp1 Lysin (Streptococcus) | (2IU_A) | | 133 (FTH**D**N) | 7 | 12 |
| LysK (Staphylococcus) | (YP_024461) | | 60 (LIT**D**Y) und 67 (WLT**D**N) | 16 | 18 |
| Ply187 (Staphylococcus) | (CAA69022) | | 596 (YAT**D**I) | 23 | 20 |
| Lysostaphin (Staphylococcus) | (AAB53783) | | | 9 | 51 |
| Ply511 (Listeria) | (Q38653) | | | 6 | 6 |
| Ply118 (Listeria) | EP0781349 B1; SEQ ID NO: 4 | 262(GTP**R**) | 129 (YGT**D**T) | 8 | 6 |
| PlyA500 (Listeria) | (AAY52812) | | | 12 | 12 |
| Ply21 (Bacillus) | (CAA72267) | 82 (G**R**G) | | 15 | 13 |
| PlyBA (Bacillus) | (CAA72266) | | | 11 | 25 |
| Ply12 (Bacillus) | (CAA72264) | | | 14 | 10 |
| PlyCD119 (Clostridium) | Neu | 87 (G**R**G) | 214 (LVT**D**I) | 7 | 13 |
| Ply3626 (Clostridium) | US 7,371,375 B2; SEQ ID NO:2 | | | 17 | 27 |

### Beispiel 10: Bestimmung von Clostripain-Schnittstellen in Listeria-Endolysinen

Wie aus Tabelle 6 ersichtlich ist, sind potentielle Schnittstellen für Clostripain in Endolysinen häufig in größerer Anzahl vorhanden (6 bis 23 in den beschriebenen Beispielen). Da eine Substitution aller potentiellen Schnittstellen die Aktivität des Endolysins negativ beeinflussen kann, ist es sinnvoll, die für die Protease zugänglichen Schnittstellen zu bestimmen und nur diese zu modifizieren. Das Listeriaendolysin Ply511 enthält sechs potentielle Schnittstellen für Clostripain. Es wurde ein Clostripainverdau von Ply511 durchgeführt, um die für Clostripain sensitiven Bereiche des Endolysins zu bestimmen. Ply511 (0,1 mg/ml) wurde für 3 h bzw. über Nacht bei Raumtemperatur mit 5 Units Clostripain (Unitdefinition nach Herstellerangaben, Sigma) verdaut in 60 µl Probenvolumen mit folgender Zusammensetzung: 25 mM Natrium Phosphate, 1 mM Calcium acetate, 2,5 mM DTT, pH 7,6. Die entstandenen Proteinfragmente wurden per SDS-Gelelektrophorese aufgetrennt (Gradientengele 10 - 20 % Acrylamid). Es entstanden 3 Banden (Molkulargewichte ca. 25 kDa, ca. 14 kDa, ca. 10 kDa), die auf PVDF-Membranen geblottet, danach ausgeschnitten und mittels Edman-Abbau N-terminal ansequenziert wurden.

Es ergaben sich folgende N-terminale Sequenzen für die Fragmente:
1. (M)V K Y T V E N K; das N-terminale Methionin war zum Teil abgespalten
2. DKLAK
3. TSNATTF

Dieses Ergebnis zeigt, dass von den sechs potentiellen Clostripainschnittstellen (R46, R62, R92, R221, R312, R326) zwei von der Protease erkannt werden, nämlich R92 und R221. Erfindungsgemäß stabilisierte Varianten von Ply511 besitzen an diesen Positionen Austausche von R zu anderen Aminosäureresten, insbesondere R62K oder R62A sowie R22 1 K oder R221A.

### Beispiel 11: Proteaseabbau eines therapeutisch einsetzbaren Proteins in verschiedenen Organen und Bestimmung der proteasesensitiven Bereiche

Bei Pharmakokinetik Studien wurde festgestellt, dass das gegen Staphylokokken eingesetzte Endolysin CHAP-Amipitti26_CBDUSA-Add2 (L56H, L57T, E164A, R168A, Y201H) in unterschiedlichen Organen unterschiedlich schnell nicht mehr nachweisbar war. Um zu testen, ob dies auf Proteaseabbau zurückzuführen war, wurde das Endolysin mit Organextrakten inkubiert und potentielle Abbaubanden auf SDS-Gelen analysiert. Gewebeproben aus Leber, Herz, Milz, Niere und Lunge von Ratten wurden tiefgefroren, wieder aufgetaut und im gleichen Volumen PBS-Puffer homogenisiert. Pro Testansatz wurden 40 µl CHAP-Amipitti26_CBDUSA-Add2 (L56H, L57T, E164A, R168A, Y201H) (1 mg/ml in 20 mM Tris, pH 8,0) und 10 µl Organextrakt (1:20 verdünnt mit PBS) gemischt und für 18 h bei Raumtemperatur bzw. bei 4 ° C inkubiert. Bei den Proteinkontrollen wurde statt Organextrakt nur PBS-Puffer zugesetzt und für dieselben Zeiten inkubiert sowie eine Proteinkontrolle ohne Inkubationszeit angesetzt. Als weitere Kontrollen wurde jeweils Organextrakt ohne Zusatz von Endolysin analysiert. Die Proben wurden mit Probenpuffer versetzt und auf 12 % igen SDS-Gelen aufgetragen.

Es zeigte sich, dass nur bei den Proben, denen Nierenextrakt zugesetzt wurde, über den Versuchszeitraum ein signifikanter Proteinabbau des Endolysins stattfand, bei allen anderen Organextrakten sowie den Kontrollen blieb das Endolysin stabil. Bei Inkubation mit dem Nierenextrakt entstanden größere Proteinfragmente mit definierter Größe, so dass es wahrscheinlich war, dass das Endolysin von einer oder wenigen nierenspezifischer Proteasen abgebaut wurde. Um die sensitiven Bereiche für diese Protease(n) zu bestimmen, wurden die entstandenen Banden (markiert mit Pfeilen und den Nummern 1 bis 7) auf eine PVDF-Membran geblottet und die Polypeptide mittels Edman-Abbau N-terminal ansequenziert. Die ungefähre Größe der entstandenen Fragmente wurde anhand einer Standardkurve abgeleitet, die aus den Laufstrecken der Banden des Molekulargewichtsstandards, bei denen das Molekulargewicht bekannt war, erstellt wurde. Es ergaben sich folgende Fragmente, die in Tabelle 7 zusammengefasst sind. Die entstandenen Proteinabschnitte wurden ausgehend von den bestimmten N-terminalen Sequenzen in Verbindung mit den Fragmentgrößen bestimmt.

**Tabelle 7: Fragmente von Endolysin CHAP-Amipitti26_CBDUSA-Add2 (L56H, L57T, E164A, R168A, Y201H) nach Abbau durch nierenspezifische Protease(n)**

| Bande Nr. | Größe (berechnet aus Laufstrecke im Gel) kDa | N-terminale Sequenz | Proteinabschnitt | Größe (berechnet aus Aminosäuresequenz) kDa |
|---|---|---|---|---|
| 1 | 58 | ASIIMEV | Komplettes Protein, N-terminales M abgespalten | 55 |
| 2 | 43 | ASIIM | N-terminales Fragment. CHAP-und Amidase Domäne. Komplette EAD bis zur Schnittstelle vor Bande 5 (ASSNTV) | 44 |
| 3 | 25 | ASKKETAPQ | Amidasedomäne. Bis zur Schnittstelle vor Bande 5 (ASSNTV). | 24 |
| 4 | 19 | ASIIMEVAT MQ | N-terminales Fragment. CHAP-Domäne bis zur Schnittstelle vor Bande 3 (ASKKETAPQ). | 19 |
| 5 | 15 | ASSNTV | C-terminales Fragment. CBD. | 12 |
| 6 | 20 | ASIIMEVAT | N-terminales Fragment. CHAP-Domäne bis zur Schnittstelle vor Bande 7 (XPTQA). | 20 |
| 7 | 41 | XPTQA | C-terminales Fragment. Amidasedomäne und CBD. | 36 |

| | | | | |
|---|---|---|---|---|
| X: Aminosäure, die nicht definiert werden konnte. | | | | |

Aus der Bestimmung der N-terminalen Sequenzen in Verbindung mit der Größenbestimmung der entstandenen Fragmente ergab sich, dass die im Nierenextrakt vorhandenen Proteasen das Endolysin CHAP-Amipitti26_CBDUSA-Add2 an 3 Positionen schneiden, nämlich nach den Aminosäuren Q171, Q175 und S384. Eine Suche nach konservierten Domänen innerhalb des Endolysins CHAP-Amipitti26_CBDUSA-Add2 in der CDD (conserved domain database) ergab 3 konservierte Domänen, nämlich N-terminal eine CHAP-Domäne (Aminosäuren 28 bis 158), gefolgt von einer Amidase_2 Domäne (Aminosäuren 199 bis 346) sowie einer C-terminal gelegenen SH3_5 Domäne (Aminosäuren 413 bis 478). Die CHAP- und Amidase 2 Domäne sind die EADs, während die SH3_5 Domäne die CBD bildet. Zwischen der CHAP und Amidase 2 Domäne liegt ein Domänenlinkerbereich (Aminosäuren 159 bis 198) sowie ein zweiter Linker zwischen der Amidase 2 Domäne und der CBD (Aminosäuren 347 bis 412). Alle drei gefundenen Schnittstellen lagen mitten in den Domänenlinkerbereichen, die sich als weniger stabil erwiesen als die konservierten Domänen. Die Proteasen aus der Niere, die für den Abbau verantwortlich waren, konnten nicht identifiziert werden, jedoch fiel auf, dass zumindest eine Protease ein Q an Position P1 benötigt sowie dass alle drei Proteaseerkennungsstellen reich an Serinen und Alaninen waren.

## Patentansprüche

1. Polypeptidvariante des Polypeptids gemäß SEQ ID NO: 1, wobei die Polypeptidvariante ausgewählt ist aus der Gruppe bestehend aus: SEQ ID NO: 2, 3, 11, 12 und 13.

2. Nukleinsäuremolekül, umfassend eine Nukleotidsequenz codierend für ein Polypeptid nach Anspruch 1.

3. Expressionsvektor, umfassend ein Nukleinsäuremolekül nach Anspruch 2.

4. Wirtszelle enthaltend ein Nukleinsäuremolekül nach Anspruch 2 oder einen Expressionsvektor nach Anspruch 3.

5. Polypeptid nach Anspruch 1 als Arzneimittel oder als Diagnostikum.

6. Polypeptid nach Anspruch 1 zur Verwendung als Arzneimittel zur Prävention oder Therapie von Erkrankungen, die durch gram positive Bakterien insbesondere Clostridien, Bazillen, Listerien, Staphylokokken, Lactobazillen, Enterokokken, Aerokokken, Pediokokken, Streptokokken, Mycoplasmen und/oder Leuconostoc hervorgerufen werden.

7. Verwendung des Polypeptids nach Anspruch 1 zur Unterdrückung des Wachstums von gram positiven Bakterien in der Umwelt-, Lebensmittel- oder Kosmetikindustrie.

8. Polypeptid nach Anspruch 1 zur Verwendung als Diagnostikum in der Medizin, Umwelt-, Lebensmittel- oder Kosmetikindustrie.

## Claims

1. Polypeptide variant of the polypeptide according to SEQ ID NO:1, wherein the polypeptide variant is selected from the group consisting of SEQ ID NO:2, 3, 11, 12, and 13.

2. Nucleic acid molecule, comprising a nucleotide sequence coding for a polypeptide according to claim 1.

3. Expression vector, comprising a nucleic acid molecule according to claim 2.

4. Host cell containing a nucleic acid molecule of claim 2 or an expression vector according to claim 3.

5. Polypeptide according to claim 1 as medicament or diagnostic agent.

6. Polypeptide according to claim 1 for use as medicament for prevention or therapy of diseases, which are caused by Gram-positive bacteria, in particular clostridia, bacilli, listeria, staphylococci, lactobacilli, entrococci, aerococci, pediococci, streptococci, mycoplasms and/or leuconostoc.

7. Use of the polypeptide according to claim 1 for inhibiting of the growth of Gram-positive bacteria in the environmental, food or cosmetic industry.

8. Polypeptide according to claim 1 for use as diagnostic agent in the medicine, environmental, food or cosmetic industry.

## Revendications

1. Variant polypeptidique du polypeptide selon SEQ ID NO :1, dans lequel le variant polypeptidique est choisi dans le groupe constitué de SEQ ID NO : 2, 3, 11, 12 et 13.

2. Molécule d'acide nucléique, comprenant une séquence nucléotidique codant pour un polypeptide selon la revendication 1.

3. Vecteur d'expression, comprenant une molécule d'acide nucléique selon la revendication 2.

4. Cellule hôte contenant une molécule d'acide nucléique de la revendication 2 ou un vecteur d'expression selon la revendication 3.

5. Polypeptide selon la revendication 1 en tant que médicament ou agent diagnostique.

6. Polypeptide selon la revendication 1 pour une utilisation en tant que médicament pour la prévention ou la thérapie de maladies causées par des bactéries Gram-positif, en particulier des clostridia, bacilli, listeria, staphylococci, lactobacilli, enterococci, aerococci, pediococci, streptococci, mycoplasmes et/ou leuconostoc.

7. Utilisation du polypeptide selon la revendication 1 pour inhiber la croissance de bactéries Gram-positif dans l'industrie environnementale, alimentaire ou cosmétique.

8. Polypeptide selon la revendication 1 pour une utilisation en tant qu'agent diagnostique dans l'industrie environnementale, alimentaire ou cosmétique.


### SEQUENCE LISTING

<110> Biomerieux S.A. Hyglos Invest GmbH
<120> Proteasestabile Zellwand lysierende Enzyme
<130> PRO-029 PCT/EP DIV 1
<140> unknown
   <141> 2012-07-13
<150> EP 07 114 785.4
   <151> 2007-08-22
<150> US 60/957 351
   <151> 2007-08-22
<150> DE 10 2007 061 929.6
   <151> 2007-12-21
<150> EP 08 152 096.7
   <151> 2008-02-28
<150> US 61/032 211
   <151> 2008-02-28
<150> DE 10 2008 023 448.6
   <151> 2008-05-14
<160> 22
<170> PatentIn version 3.3
<210> 1
   <211> 496
   <212> PRT
   <213> Artificial
<220>
   <223> Polypetide comprising substitution at R167
<400> 1
   Met Ser Ile Ile Met Glu Val Ala Thr Met Gln Ala Lys Leu Thr Lys
   1               5                   10                  15
   Lys Glu Phe Ile Glu Trp Leu Lys Thr Ser Glu Gly Lys Gln Phe Asn
               20                  25                  30
   Val Asp Leu Trp Tyr Gly Phe Gln Cys Phe Asp Tyr Ala Asn Ala Gly
           35                  40                  45
   Trp Lys Val Leu Phe Gly Leu Leu Leu Lys Gly Leu Gly Ala Lys Asp
       50                  55                  60
   Ile Pro Phe Ala Asn Asn Phe Asp Gly Leu Ala Thr Val Tyr Gln Asn
   65                  70                  75                  80
   Thr Pro Asp Phe Leu Ala Gln Pro Gly Asp Met Val Val Phe Gly Ser
                   85                  90                  95
   Asn Tyr Gly Ala Gly Tyr Gly His Val Ala Trp Val Ile Glu Ala Thr
               100                 105                 110
   Leu Asp Tyr Ile Ile Val Tyr Glu Gln Asn Trp Leu Gly Gly Gly Trp
           115                 120                 125
   Thr Asp Arg Ile Glu Gln Pro Gly Trp Gly Trp Glu Lys Val Thr Arg
       130                 135                 140
   Arg Gln His Ala Tyr Asp Phe Pro Met Trp Phe Ile Arg Pro Asn Phe
   145                 150                 155                 160
   Lys Ser Glu Thr Ala Pro Ala Ser Ile Gln Ser Pro Thr Gln Ala Ser
                   165                 170                 175
   Lys Lys Glu Thr Ala Lys Pro Gln Pro Lys Ala Val Glu Leu Lys Ile
               180                 185                 190
   Ile Lys Asp Val Val Lys Gly Tyr Asp Leu Pro Lys Arg Gly Gly Asn
           195                 200                 205
   Pro Lys Gly Ile Val Ile His Asn Asp Ala Gly Ser Lys Gly Ala Thr
       210                 215                 220
   Ala Glu Ala Tyr Arg Asn Gly Leu Val Asn Ala Pro Ser Ser Arg Leu
   225                 230                 235                 240
   Glu Ala Gly Ile Ala His Ser Tyr Val Ser Gly Asn Thr Val Trp Gln
                   245                 250                 255
   Ala Leu Asp Glu Ser Gln Val Gly Trp His Thr Ala Asn Gln Leu Gly
               260                 265                 270
   Asn Lys Tyr Tyr Tyr Gly Ile Glu Val Cys Gln Ser Met Gly Ala Asp
           275                 280                 285
   Asn Ala Thr Phe Leu Lys Asn Glu Gln Ala Thr Phe Gln Glu Cys Ala
       290                 295                 300
   Arg Leu Leu Lys Lys Trp Gly Leu Pro Ala Asn Arg Asn Thr Ile Arg
   305                 310                 315                 320
   Leu His Asn Glu Phe Thr Ser Thr Ser Cys Pro His Arg Ser Ser Val
                   325                 330                 335
   Leu His Thr Gly Phe Asp Pro Val Thr Arg Gly Leu Leu Pro Glu Asp
               340                 345                 350
   Lys Arg Leu Gln Leu Lys Asp Tyr Phe Ile Lys Gln Ile Arg Ala Tyr
   355 360 365
   Met Asp Gly Lys Ile Pro Val Ala Thr Val Ser Asn Glu Ser Ser Ala
   370 375 380
   Ser Ser Asn Thr Val Lys Pro Val Ala Glu Leu Met Pro Pro Val Pro
   385 390 395 400
   Ala Gly Tyr Thr Leu Asp Lys Asn Asn Val Pro Tyr Lys Lys Glu Gln
   405 410 415
   Gly Asn Tyr Thr Val Ala Asn Val Lys Gly Asn Asn Val Arg Asp Gly
   420 425 430
   Tyr Ser Thr Asn Ser Arg Ile Thr Gly Val Leu Pro Asn Asn Thr Thr
   435 440 445
   Ile Thr Tyr Asp Gly Ala Tyr Cys Ile Asn Gly Tyr Arg Trp Ile Thr
   450 455 460
   Tyr Ile Ala Asn Ser Gly Gln Arg Arg Tyr Ile Ala Thr Gly Glu Val
   465 470 475 480
   Asp Ile Ala Gly Asn Arg Ile Ser Ser Phe Gly Lys Phe Ser Ala Val
   485 490 495
<210> 2
   <211> 496
   <212> PRT
   <213> Artificial
<220>
   <223> Polypetide comprising substitution at E163 and R167
<400> 2
   Met Ser Ile Ile Met Glu Val Ala Thr Met Gln Ala Lys Leu Thr Lys
   1 5 10 15
   Lys Glu Phe Ile Glu Trp Leu Lys Thr Ser Glu Gly Lys Gln Phe Asn
   20 25 30
   Val Asp Leu Trp Tyr Gly Phe Gln Cys Phe Asp Tyr Ala Asn Ala Gly
   35 40 45
   Trp Lys Val Leu Phe Gly Leu Leu Leu Lys Gly Leu Gly Ala Lys Asp
   50 55 60
   Ile Pro Phe Ala Asn Asn Phe Asp Gly Leu Ala Thr Val Tyr Gln Asn
   65                  70                  75                  80
   Thr Pro Asp Phe Leu Ala Gln Pro Gly Asp Met Val Val Phe Gly Ser
                   85                  90                  95
   Asn Tyr Gly Ala Gly Tyr Gly His Val Ala Trp Val Ile Glu Ala Thr
               100                 105                 110
   Leu Asp Tyr Ile Ile Val Tyr Glu Gln Asn Trp Leu Gly Gly Gly Trp
           115                 120                 125
   Thr Asp Arg Ile Glu Gln Pro Gly Trp Gly Trp Glu Lys Val Thr Arg
       130                 135                 140
   Arg Gln His Ala Tyr Asp Phe Pro Met Trp Phe Ile Arg Pro Asn Phe
   145                 150                 155                 160
   Lys Ser Ala Thr Ala Pro Ala Ser Ile Gln Ser Pro Thr Gln Ala Ser
                   165                 170                 175
   Lys Lys Glu Thr Ala Lys Pro Gln Pro Lys Ala Val Glu Leu Lys Ile
               180                 185                 190
   Ile Lys Asp Val Val Lys Gly Tyr Asp Leu Pro Lys Arg Gly Gly Asn
           195                 200                 205
   Pro Lys Gly Ile Val Ile His Asn Asp Ala Gly Ser Lys Gly Ala Thr
       210                 215                 220
   Ala Glu Ala Tyr Arg Asn Gly Leu Val Asn Ala Pro Ser Ser Arg Leu
   225                 230                 235                 240
   Glu Ala Gly Ile Ala His Ser Tyr Val Ser Gly Asn Thr Val Trp Gln
                   245                 250                 255
   Ala Leu Asp Glu Ser Gln Val Gly Trp His Thr Ala Asn Gln Leu Gly
               260                 265                 270
   Asn Lys Tyr Tyr Tyr Gly Ile Glu Val Cys Gln Ser Met Gly Ala Asp
           275                 280                 285
   Asn Ala Thr Phe Leu Lys Asn Glu Gln Ala Thr Phe Gln Glu Cys Ala
       290                 295                 300
   Arg Leu Leu Lys Lys Trp Gly Leu Pro Ala Asn Arg Asn Thr Ile Arg
   305                 310                 315                 320
   Leu His Asn Glu Phe Thr Ser Thr Ser Cys Pro His Arg Ser Ser Val
                   325                 330                 335
   Leu His Thr Gly Phe Asp Pro Val Thr Arg Gly Leu Leu Pro Glu Asp
   340 345 350
   Lys Arg Leu Gln Leu Lys Asp Tyr Phe Ile Lys Gln Ile Arg Ala Tyr
   355 360 365
   Met Asp Gly Lys Ile Pro Val Ala Thr Val Ser Asn Glu Ser Ser Ala
   370 375 380
   Ser Ser Asn Thr Val Lys Pro Val Ala Glu Leu Met Pro Pro Val Pro
   385 390 395 400
   Ala Gly Tyr Thr Leu Asp Lys Asn Asn Val Pro Tyr Lys Lys Glu Gln
   405 410 415
   Gly Asn Tyr Thr Val Ala Asn Val Lys Gly Asn Asn Val Arg Asp Gly
   420 425 430
   Tyr Ser Thr Asn Ser Arg Ile Thr Gly Val Leu Pro Asn Asn Thr Thr
   435 440 445
   Ile Thr Tyr Asp Gly Ala Tyr Cys Ile Asn Gly Tyr Arg Trp Ile Thr
   450 455 460
   Tyr Ile Ala Asn Ser Gly Gln Arg Arg Tyr Ile Ala Thr Gly Glu Val
   465 470 475 480
   Asp Ile Ala Gly Asn Arg Ile Ser Ser Phe Gly Lys Phe Ser Ala Val
   485 490 495
<210> 3
   <211> 496
   <212> PRT
   <213> Artificial
<220>
   <223> Polypetide comprising substitution at E163, R167, E179, E189
<210> 4
   <211> 271
   <212> PRT
   <213> Artificial
<220>
   <223> Polypetide comprising substitution at D214
<400> 4
   Met Lys Ile Gly Ile Asn Cys Gly His Thr Lys Thr Gly Ala Gly Ser
   1 5 10 15
   Gly Ala Ile Gly Lys Ile Asn Glu Ser Ile Glu Thr Arg Asn Val Gly
   20 25 30
   Tyr Lys Val Ile Asp Lys Leu Lys Lys Leu Gly Asn Asn Val Val Asp
               35                  40                  45
   Cys Thr Ile Asp Lys Ala Ser Thr Gln Ser Glu Cys Leu Ser Lys Ile
       50                  55                  60
   Thr Ala Gln Ala Asn Arg Gln Asp Leu Asp Trp Phe Ile Ser Ile His
   65                  70                  75                  80
   Phe Asn Ala Gly Gly Gly Lys Gly Cys Glu Val Tyr Thr Tyr Lys Gly
                   85                  90                  95
   Lys Gln Tyr Gln Asp Ala Ile Asp Val Cys Lys Lys Ile Ser Asp Leu
               100                 105                 110
   Gly Phe Thr Asn Arg Gly Val Lys Asp Gly Ser Gly Leu Tyr Val Val
           115                 120                 125
   Lys Lys Thr Lys Ala Lys Ser Met Leu Ile Glu Val Cys Phe Val Asp
       130                 135                 140
   Thr Glu Asp Ala Asn Lys Tyr Leu Ser Leu Gly Ala Asp Lys Leu Ala
   145                 150                 155                 160
   Thr Ala Ile Val Glu Ala Ile Thr Lys His Ile Ser Ser Ala Glu Glu
                   165                 170                 175
   Asn Asn Tyr Asn Arg Tyr Lys His Thr Ile Val Tyr Ser Gly Asp Asp
               180                 185                 190
   Lys Val Ser Ala Asp Ile Leu Gly Leu Tyr Tyr Lys Arg Lys Lys Glu
           195                 200                 205
   Ser Tyr Leu Val Thr Glu Ile Lys Asp Tyr Lys Pro His Arg Thr Gln
       210                 215                 220
   Asn Leu Tyr Val Ile Gly Gly Val Thr Cys Asn Lys Met Lys Glu Met
   225                 230                 235                 240
   Ser Lys Thr Thr Gly Glu Lys Phe Thr Gln Leu Tyr Gly Asn Asp Val
                   245                 250                 255
   Trp Ser Thr Met Asp Lys Ala Ile Glu Phe Val Lys Glu Lys Leu
               260                 265                 270
<210> 5
   <211> 271
   <212> PRT
   <213> Artificial
<220>
   <223> Polypetide comprising substitution at R87 and D214
<400> 5
   Met Lys Ile Gly Val Asn Cys Gly His Thr Lys Thr Gly Ala Gly Ser
   1               5                   10                  15
   Gly Ala Ile Gly Lys Ile Asn Glu Ser Ile Glu Thr Arg Asn Val Gly
               20                  25                  30
   Tyr Lys Val Ile Asp Lys Leu Lys Thr Leu Gly Asn Asn Val Val Asp
           35                  40                  45
   Cys Thr Ile Asp Lys Ala Ser Thr Gln Ser Glu Cys Leu Ser Lys Ile
       50                  55                  60
   Ala Thr Gln Ala Asn Arg Gln Asp Leu Asp Trp Phe Ile Ser Ile His
   65                  70                  75                  80
   Phe Asn Ala Gly Lys Gly Lys Gly Cys Glu Val Tyr Thr Tyr Lys Gly
                   85                  90                  95
   Lys Gln Tyr Gln Asp Ala Ile Asp Val Cys Lys Lys Ile Ser Asp Leu
               100                 105                 110
   Gly Phe Thr Asn Arg Gly Val Lys Asp Gly Ser Gly Leu Tyr Val Val
           115                 120                 125
   Lys Lys Thr Lys Ala Lys Ser Met Leu Ile Glu Val Cys Phe Val Asp
       130                 135                 140
   Ser Glu Asp Ala Asn Lys Tyr Leu Ser Leu Gly Ala Asp Lys Leu Ala
   145                 150                 155                 160
   Thr Ala Ile Val Glu Ala Ile Thr Lys His Ile Ser Ser Ala Glu Glu
                   165                 170                 175
   Asn Asn Tyr Asn Arg Tyr Lys His Thr Ile Val Tyr Ser Gly Asp Asp
               180                 185                 190
   Lys Val Ser Ala Asp Ile Leu Gly Leu Tyr Tyr Lys Arg Lys Lys Glu
           195                 200                 205
   Ser Tyr Leu Val Thr Glu Ile Lys Asp Tyr Lys Pro His Arg Thr Gln
       210                 215                 220
   Asn Leu Tyr Val Ile Gly Gly Val Thr Cys Asn Lys Met Lys Glu Met
   225                 230                 235                 240
   Ser Lys Thr Thr Gly Glu Lys Phe Thr Gln Leu Tyr Gly Asn Asp Val
   245 250 255
   Trp Ser Thr Met Asp Lys Ala Ile Glu Phe Val Lys Glu Lys Leu
   260 265 270
<210> 6
   <211> 341
   <212> PRT
   <213> Artificial
<220>
   <223> Polypeptide comprising substitution at E7 and E40
<400> 6
   Met Val Lys Tyr Thr Val Ala Asn Lys Ile Ile Ala Gly Leu Pro Lys
   1 5 10 15
   Gly Lys Leu Lys Gly Ala Asn Phe Val Ile Ala His Glu Thr Ala Asn
   20 25 30
   Ser Lys Ser Thr Ile Asp Asn Gln Val Ser Tyr Met Thr Arg Asn Trp
   35 40 45
   Lys Asn Ala Phe Val Thr His Phe Val Gly Gly Gly Gly Arg Val Val
   50 55 60
   Gln Val Ala Asn Val Asn Tyr Val Ser Trp Gly Ala Gly Gln Tyr Ala
   65 70 75 80
   Asn Ser Tyr Ser Tyr Ala Gln Val Glu Leu Cys Arg Thr Ser Asn Ala
   85 90 95
   Thr Thr Phe Lys Lys Asp Tyr Glu Val Tyr Cys Gln Leu Leu Val Asp
   100 105 110
   Leu Ala Lys Lys Ala Gly Ile Pro Ile Thr Leu Asp Ser Gly Ser Lys
   115 120 125
   Thr Ser Asp Lys Gly Ile Lys Ser His Lys Trp Val Ala Asp Lys Leu
   130 135 140
   Gly Gly Thr Thr His Gln Asp Pro Tyr Ala Tyr Leu Ser Ser Trp Gly
   145 150 155 160
   Ile Ser Lys Ala Gln Phe Ala Ser Asp Leu Ala Lys Val Ser Gly Gly
   165 170 175
   Gly Asn Thr Gly Thr Ala Pro Ala Lys Pro Ser Thr Pro Ala Pro Lys
   180 185 190
   Pro Ser Thr Pro Ser Thr Asn Leu Asp Lys Leu Gly Leu Val Asp Tyr
   195 200 205
   Met Asn Ala Lys Lys Met Asp Ser Ser Tyr Ser Asn Arg Asp Lys Leu
   210 215 220
   Ala Lys Gln Tyr Gly Ile Ala Asn Tyr Ser Gly Thr Ala Ser Gln Asn
   225 230 235 240
   Thr Thr Leu Leu Ser Lys Ile Lys Gly Gly Ala Pro Lys Pro Ser Thr
   245 250 255
   Pro Ala Pro Lys Pro Ser Thr Ser Thr Ala Lys Lys Ile Tyr Phe Pro
   260 265 270
   Pro Asn Lys Gly Asn Trp Ser Val Tyr Pro Thr Asn Lys Ala Pro Val
   275 280 285
   Lys Ala Asn Ala Ile Gly Ala Ile Asn Pro Thr Lys Phe Gly Gly Leu
   290 295 300
   Thr Tyr Thr Ile Gln Lys Asp Arg Gly Asn Gly Val Tyr Glu Ile Gln
   305 310 315 320
   Thr Asp Gln Phe Gly Arg Val Gln Val Tyr Gly Ala Pro Ser Thr Gly
   325 330 335
   Ala Val Ile Lys Lys
   340
<210> 7
   <211> 341
   <212> PRT
   <213> artificial
<220>
   <223> Polypeptide comprising substitution at R92K, R221K
<400> 7
   Met Val Lys Tyr Thr Val Glu Asn Lys Ile Ile Ala Gly Leu Pro Lys
   1               5                   10                  15
   Gly Lys Leu Lys Gly Ala Asn Phe Val Ile Ala His Glu Thr Ala Asn
               20                  25                  30
   Ser Lys Ser Thr Ile Asp Asn Glu Val Ser Tyr Met Thr Arg Asn Trp
           35                  40                  45
   Lys Asn Ala Phe Val Thr His Phe Val Gly Gly Gly Gly Arg Val Val
       50                  55                  60
   Gln Val Ala Asn Val Asn Tyr Val Ser Trp Gly Ala Gly Gln Tyr Ala
   65                  70                  75                  80
   Asn Ser Tyr Ser Tyr Ala Gln Val Glu Leu Cys Lys Thr Ser Asn Ala
                   85                  90                  95
   Thr Thr Phe Lys Lys Asp Tyr Glu Val Tyr Cys Gln Leu Leu Val Asp
               100                 105                 110
   Leu Ala Lys Lys Ala Gly Ile Pro Ile Thr Leu Asp Ser Gly Ser Lys
           115                 120                 125
   Thr Ser Asp Lys Gly Ile Lys Ser His Lys Trp Val Ala Asp Lys Leu
       130                 135                 140
   Gly Gly Thr Thr His Gln Asp Pro Tyr Ala Tyr Leu Ser Ser Trp Gly
   145                 150                 155                 160
   Ile Ser Lys Ala Gln Phe Ala Ser Asp Leu Ala Lys Val Ser Gly Gly
                   165                 170                 175
   Gly Asn Thr Gly Thr Ala Pro Ala Lys Pro Ser Thr Pro Ala Pro Lys
               180                 185                 190
   Pro Ser Thr Pro Ser Thr Asn Leu Asp Lys Leu Gly Leu Val Asp Tyr
           195                 200                 205
   Met Asn Ala Lys Lys Met Asp Ser Ser Tyr Ser Asn Lys Asp Lys Leu
       210                 215                 220
   Ala Lys Gln Tyr Gly Ile Ala Asn Tyr Ser Gly Thr Ala Ser Gln Asn
   225                 230                 235                 240
   Thr Thr Leu Leu Ser Lys Ile Lys Gly Gly Ala Pro Lys Pro Ser Thr
                   245                 250                 255
   Pro Ala Pro Lys Pro Ser Thr Ser Thr Ala Lys Lys Ile Tyr Phe Pro
               260                 265                 270
   Pro Asn Lys Gly Asn Trp Ser Val Tyr Pro Thr Asn Lys Ala Pro Val
           275                 280                 285
   Lys Ala Asn Ala Ile Gly Ala Ile Asn Pro Thr Lys Phe Gly Gly Leu
       290                 295                 300
   Thr Tyr Thr Ile Gln Lys Asp Arg Gly Asn Gly Val Tyr Glu Ile Gln
   305                 310                 315                 320
   Thr Asp Gln Phe Gly Arg Val Gln Val Tyr Gly Ala Pro Ser Thr Gly
   325 330 335
   Ala Val Ile Lys Lys
   340
<210> 8
   <211> 341
   <212> PRT
   <213> artificial
<220>
   <223> Polypeptide comprising substitution at R92A, R221A
<400> 8
   Met Val Lys Tyr Thr Val Glu Asn Lys Ile Ile Ala Gly Leu Pro Lys
   1               5                   10                  15
   Gly Lys Leu Lys Gly Ala Asn Phe Val Ile Ala His Glu Thr Ala Asn
               20                  25                  30
   Ser Lys Ser Thr Ile Asp Asn Glu Val Ser Tyr Met Thr Arg Asn Trp
           35                  40                  45
   Lys Asn Ala Phe Val Thr His Phe Val Gly Gly Gly Gly Arg Val Val
       50                  55                  60
   Gln Val Ala Asn Val Asn Tyr Val Ser Trp Gly Ala Gly Gln Tyr Ala
   65                  70                  75                  80
   Asn Ser Tyr Ser Tyr Ala Gln Val Glu Leu Cys Ala Thr Ser Asn Ala
                   85                  90                  95
   Thr Thr Phe Lys Lys Asp Tyr Glu Val Tyr Cys Gln Leu Leu Val Asp
               100                 105                 110
   Leu Ala Lys Lys Ala Gly Ile Pro Ile Thr Leu Asp Ser Gly Ser Lys
           115                 120                 125
   Thr Ser Asp Lys Gly Ile Lys Ser His Lys Trp Val Ala Asp Lys Leu
       130                 135                 140
   Gly Gly Thr Thr His Gln Asp Pro Tyr Ala Tyr Leu Ser Ser Trp Gly
   145                 150                 155                 160
   Ile Ser Lys Ala Gln Phe Ala Ser Asp Leu Ala Lys Val Ser Gly Gly
                   165                 170                 175
   Gly Asn Thr Gly Thr Ala Pro Ala Lys Pro Ser Thr Pro Ala Pro Lys
               180                 185                 190
   Pro Ser Thr Pro Ser Thr Asn Leu Asp Lys Leu Gly Leu Val Asp Tyr
   195 200 205
   Met Asn Ala Lys Lys Met Asp Ser Ser Tyr Ser Asn Ala Asp Lys Leu
   210 215 220
   Ala Lys Gln Tyr Gly Ile Ala Asn Tyr Ser Gly Thr Ala Ser Gln Asn
   225 230 235 240
   Thr Thr Leu Leu Ser Lys Ile Lys Gly Gly Ala Pro Lys Pro Ser Thr
   245 250 255
   Pro Ala Pro Lys Pro Ser Thr Ser Thr Ala Lys Lys Ile Tyr Phe Pro
   260 265 270
   Pro Asn Lys Gly Asn Trp Ser Val Tyr Pro Thr Asn Lys Ala Pro Val
   275 280 285
   Lys Ala Asn Ala Ile Gly Ala Ile Asn Pro Thr Lys Phe Gly Gly Leu
   290 295 300
   Thr Tyr Thr Ile Gln Lys Asp Arg Gly Asn Gly Val Tyr Glu Ile Gln
   305 310 315 320
   Thr Asp Gln Phe Gly Arg Val Gln Val Tyr Gly Ala Pro Ser Thr Gly
   325 330 335
   Ala Val Ile Lys Lys
   340
<210> 9
   <211> 317
   <212> PRT
   <213> artificial
<220>
   <223> Polypeptide comprising substitution at D112E
<400> 9
   Met Ala Tyr Lys Val Glu Arg Arg Ile Arg Pro Gly Leu Pro Gln Val
   1               5                   10                  15
   Gly Tyr Ala Pro Tyr Gly Gln Val His Ala His Ser Thr Gly Asn Pro
               20                  25                  30
   Arg Ser Thr Ala Gln Asn Glu Ala Asp Tyr Phe Gln Thr Lys Asp Ile
           35                  40                  45
   Thr Thr Gly Phe Tyr Thr His Leu Val Gly Asn Gly Arg Val Ile Gln
       50                  55                  60
   Leu Ala Glu Val Asn Arg Gly Ala Trp Asp Val Gly Gly Gly Trp Asn
   65 70 75 80
   Gln Trp Gly Tyr Ala Ser Val Glu Leu Ile Glu Ser His Ser Asn Arg
   85 90 95
   Asp Glu Phe Met Arg Asp Tyr Lys Ile Tyr Cys Glu Leu Leu His Glu
   100 105 110
   Leu Ala Lys Gln Ala Gly Leu Pro Thr Thr Val Asp Gln Gly Asn Thr
   115 120 125
   Gly Ile Ile Thr His Asn Tyr Ala Thr His Asn Gln Pro Asn Asn Gly
   130 135 140
   Ser Asp His Val Asp Pro Ile Pro Tyr Leu Ala Lys Trp Gly Ile Asn
   145 150 155 160
   Leu Ala Gln Phe Arg Ser Asp Val Ala Asn Ala Lys Ser Asn Ser Lys
   165 170 175
   Pro Val Thr Pro Ser Lys Pro Val Ser His Asp Lys Ala Ile Ala Lys
   180 185 190
   Ser Pro Ala Lys Thr Val Asn Gly Tyr Thr Gly Lys Met Asp Lys Phe
   195 200 205
   Asn Val Gln Gly Asn Lys Phe Arg Val Ala Gly Trp Met Leu Pro Thr
   210 215 220
   Ala Gly Gly Gln Pro Tyr Asn Tyr Gly Tyr Val Phe Leu Leu Asp Ala
   225 230 235 240
   Lys Thr Gly Lys Glu Ile Ala Arg Gln Leu Ala Gly Ala Val Ser Arg
   245 250 255
   Pro Asp Val Thr Lys Ala Tyr Gly Val Lys Gly Gly Thr Asn Tyr Gly
   260 265 270
   Leu Asp Val Thr Phe Asp Val Lys Lys Leu Lys Gly Lys Lys Phe Tyr
   275 280 285
   Ala Met Phe Arg Arg Thr Asn Asp Lys Ala Gly Asn Thr Ala Gly Gly
   290 295 300
   His Lys Asp Ile Gly Phe Asn Glu Phe Tyr Phe Thr Leu
   305 310 315
<210> 10
   <211> 317
   <212> PRT
   <213> artificial
<220>
   <223> Polypeptide comprising substitution at D112A
<400> 10
   Met Ala Tyr Lys Val Glu Arg Arg Ile Arg Pro Gly Leu Pro Gln Val
   1               5                   10                  15
   Gly Tyr Ala Pro Tyr Gly Gln Val His Ala His Ser Thr Gly Asn Pro
               20                  25                  30
   Arg Ser Thr Ala Gln Asn Glu Ala Asp Tyr Phe Gln Thr Lys Asp Ile
           35                  40                  45
   Thr Thr Gly Phe Tyr Thr His Leu Val Gly Asn Gly Arg Val Ile Gln
       50                  55                  60
   Leu Ala Glu Val Asn Arg Gly Ala Trp Asp Val Gly Gly Gly Trp Asn
   65                  70                  75                  80
   Gln Trp Gly Tyr Ala Ser Val Glu Leu Ile Glu Ser His Ser Asn Arg
                   85                  90                  95
   Asp Glu Phe Met Arg Asp Tyr Lys Ile Tyr Cys Glu Leu Leu His Ala
               100                 105                 110
   Leu Ala Lys Gln Ala Gly Leu Pro Thr Thr Val Asp Gln Gly Asn Thr
           115                 120                 125
   Gly Ile Ile Thr His Asn Tyr Ala Thr His Asn Gln Pro Asn Asn Gly
       130                 135                 140
   Ser Asp His Val Asp Pro Ile Pro Tyr Leu Ala Lys Trp Gly Ile Asn
   145                 150                 155                 160
   Leu Ala Gln Phe Arg Ser Asp Val Ala Asn Ala Lys Ser Asn Ser Lys
                   165                 170                 175
   Pro Val Thr Pro Ser Lys Pro Val Ser His Asp Lys Ala Ile Ala Lys
               180                 185                 190
   Ser Pro Ala Lys Thr Val Asn Gly Tyr Thr Gly Lys Met Asp Lys Phe
           195                 200                 205
   Asn Val Gln Gly Asn Lys Phe Arg Val Ala Gly Trp Met Leu Pro Thr
       210                 215                 220
   Ala Gly Gly Gln Pro Tyr Asn Tyr Gly Tyr Val Phe Leu Leu Asp Ala
   225 230 235 240
   Lys Thr Gly Lys Glu Ile Ala Arg Gln Leu Ala Gly Ala Val Ser Arg
   245 250 255
   Pro Asp Val Thr Lys Ala Tyr Gly Val Lys Gly Gly Thr Asn Tyr Gly
   260 265 270
   Leu Asp Val Thr Phe Asp Val Lys Lys Leu Lys Gly Lys Lys Phe Tyr
   275 280 285
   Ala Met Phe Arg Arg Thr Asn Asp Lys Ala Gly Asn Thr Ala Gly Gly
   290 295 300
   His Lys Asp Ile Gly Phe Asn Glu Phe Tyr Phe Thr Leu
   305 310 315
<210> 11
   <211> 496
   <212> PRT
   <213> artificial
<220>
   <223> Polypetide comprising substitution at E163, R167
<400> 11
   Met Ser Ile Ile Met Glu Val Ala Thr Met Gln Ala Lys Leu Thr Lys
   1 5 10 15
   Lys Glu Phe Ile Glu Trp Leu Lys Thr Ser Glu Gly Lys Gln Phe Asn
   20 25 30
   Val Asp Leu Trp Tyr Gly Phe Gln Cys Phe Asp Tyr Ala Asn Ala Gly
   35 40 45
   Trp Lys Val Leu Phe Gly Leu Leu Leu Lys Gly Leu Gly Ala Lys Asp
   50 55 60
   Ile Pro Phe Ala Asn Asn Phe Asp Gly Leu Ala Thr Val Tyr Gln Asn
   65 70 75 80
   Thr Pro Asp Phe Leu Ala Gln Pro Gly Asp Met Val Val Phe Gly Ser
   85 90 95
   Asn Tyr Gly Ala Gly Tyr Gly His Val Ala Trp Val Ile Glu Ala Thr
   100 105 110
   Leu Asp Tyr Ile Ile Val Tyr Glu Gln Asn Trp Leu Gly Gly Gly Trp
   115 120 125
   Thr Asp Arg Ile Glu Gln Pro Gly Trp Gly Trp Glu Lys Val Thr Arg
   130 135 140
   Arg Gln His Ala Tyr Asp Phe Pro Met Trp Phe Ile Arg Pro Asn Phe
   145 150 155 160
   Lys Ser Gln Thr Ala Pro Ala Ser Ile Gln Ser Pro Thr Gln Ala Ser
   165 170 175
   Lys Lys Glu Thr Ala Lys Pro Gln Pro Lys Ala Val Glu Leu Lys Ile
   180 185 190
   Ile Lys Asp Val Val Lys Gly Tyr Asp Leu Pro Lys Arg Gly Gly Asn
   195 200 205
   Pro Lys Gly Ile Val Ile His Asn Asp Ala Gly Ser Lys Gly Ala Thr
   210 215 220
   Ala Glu Ala Tyr Arg Asn Gly Leu Val Asn Ala Pro Ser Ser Arg Leu
   225 230 235 240
   Glu Ala Gly Ile Ala His Ser Tyr Val Ser Gly Asn Thr Val Trp Gln
   245 250 255
   Ala Leu Asp Glu Ser Gln Val Gly Trp His Thr Ala Asn Gln Leu Gly
   260 265 270
   Asn Lys Tyr Tyr Tyr Gly Ile Glu Val Cys Gln Ser Met Gly Ala Asp
   275 280 285
   Asn Ala Thr Phe Leu Lys Asn Glu Gln Ala Thr Phe Gln Glu Cys Ala
   290 295 300
   Arg Leu Leu Lys Lys Trp Gly Leu Pro Ala Asn Arg Asn Thr Ile Arg
   305 310 315 320
   Leu His Asn Glu Phe Thr Ser Thr Ser Cys Pro His Arg Ser Ser Val
   325 330 335
   Leu His Thr Gly Phe Asp Pro Val Thr Arg Gly Leu Leu Pro Glu Asp
   340 345 350
   Lys Arg Leu Gln Leu Lys Asp Tyr Phe Ile Lys Gln Ile Arg Ala Tyr
   355 360 365
   Met Asp Gly Lys Ile Pro Val Ala Thr Val Ser Asn Glu Ser Ser Ala
   370 375 380
   Ser Ser Asn Thr Val Lys Pro Val Ala Glu Leu Met Pro Pro Val Pro
   385 390 395 400
   Ala Gly Tyr Thr Leu Asp Lys Asn Asn Val Pro Tyr Lys Lys Glu Gln
   405 410 415
   Gly Asn Tyr Thr Val Ala Asn Val Lys Gly Asn Asn Val Arg Asp Gly
   420 425 430
   Tyr Ser Thr Asn Ser Arg Ile Thr Gly Val Leu Pro Asn Asn Thr Thr
   435 440 445
   Ile Thr Tyr Asp Gly Ala Tyr Cys Ile Asn Gly Tyr Arg Trp Ile Thr
   450 455 460
   Tyr Ile Ala Asn Ser Gly Gln Arg Arg Tyr Ile Ala Thr Gly Glu Val
   465 470 475 480
   Asp Ile Ala Gly Asn Arg Ile Ser Ser Phe Gly Lys Phe Ser Ala Val
   485 490 495
<210> 12
   <211> 497
   <212> PRT
   <213> artificial
<220>
   <223> Polypeptide comprising substitutions at L56, L57, E164, R168, Y201, Q171, Q175, S384
<400> 12
   Met Ala Ser Ile Ile Met Glu Val Ala Thr Met Gln Ala Lys Leu Thr
   1 5 10 15
   Lys Lys Glu Phe Ile Glu Trp Leu Lys Thr Ser Glu Gly Lys Gln Phe
   20 25 30
   Asn Val Asp Leu Trp Tyr Gly Phe Gln Cys Phe Asp Tyr Ala Asn Ala
   35 40 45
   Gly Trp Lys Val Leu Phe Gly His Thr Leu Lys Gly Leu Gly Ala Lys
   50 55 60
   Asp Ile Pro Phe Ala Asn Asn Phe Asp Gly Leu Ala Thr Val Tyr Gln
   65 70 75 80
   Asn Thr Pro Asp Phe Leu Ala Gln Pro Gly Asp Met Val Val Phe Gly
   85 90 95
   Ser Asn Tyr Gly Ala Gly Tyr Gly His Val Ala Trp Val Ile Glu Ala
   100 105 110
   Thr Leu Asp Tyr Ile Ile Val Tyr Glu Gln Asn Trp Leu Gly Gly Gly
   115 120 125
   Trp Thr Asp Arg Ile Glu Gln Pro Gly Trp Gly Trp Glu Lys Val Thr
   130 135 140
   Arg Arg Gln His Ala Tyr Asp Phe Pro Met Trp Phe Ile Arg Pro Asn
   145 150 155 160
   Phe Lys Ser Ala Thr Ala Pro Ala Ser Ile Asn Ser Pro Thr Asn Ala
   165 170 175
   Ser Lys Lys Glu Thr Ala Lys Pro Gln Pro Lys Ala Val Glu Leu Lys
   180 185 190
   Ile Ile Lys Asp Val Val Lys Gly His Asp Leu Pro Lys Arg Gly Gly
   195 200 205
   Asn Pro Lys Gly Ile Val Ile His Asn Asp Ala Gly Ser Lys Gly Ala
   210 215 220
   Thr Ala Glu Ala Tyr Arg Asn Gly Leu Val Asn Ala Pro Ser Ser Arg
   225 230 235 240
   Leu Glu Ala Gly Ile Ala His Ser Tyr Val Ser Gly Asn Thr Val Trp
   245 250 255
   Gln Ala Leu Asp Glu Ser Gln Val Gly Trp His Thr Ala Asn Gln Leu
   260 265 270
   Gly Asn Lys Tyr Tyr Tyr Gly Ile Glu Val Cys Gln Ser Met Gly Ala
   275 280 285
   Asp Asn Ala Thr Phe Leu Lys Asn Glu Gln Ala Thr Phe Gln Glu Cys
   290 295 300
   Ala Arg Leu Leu Lys Lys Trp Gly Leu Pro Ala Asn Arg Asn Thr Ile
   305 310 315 320
   Arg Leu His Asn Glu Phe Thr Ser Thr Ser Cys Pro His Arg Ser Ser
   325 330 335
   Val Leu His Thr Gly Phe Asp Pro Val Thr Arg Gly Leu Leu Pro Glu
   340 345 350
   Asp Lys Arg Leu Gln Leu Lys Asp Tyr Phe Ile Lys Gln Ile Arg Ala
   355 360 365
   Tyr Met Asp Gly Lys Ile Pro Val Ala Thr Val Ser Asn Glu Ser Thr
   370 375 380
   Ala Ser Ser Asn Thr Val Lys Pro Val Ala Glu Leu Met Pro Pro Val
   385 390 395 400
   Pro Ala Gly Tyr Thr Leu Asp Lys Asn Asn Val Pro Tyr Lys Lys Glu
   405 410 415
   Gln Gly Asn Tyr Thr Val Ala Asn Val Lys Gly Asn Asn Val Arg Asp
   420 425 430
   Gly Tyr Ser Thr Asn Ser Arg Ile Thr Gly Val Leu Pro Asn Asn Thr
   435 440 445
   Thr Ile Thr Tyr Asp Gly Ala Tyr Cys Ile Asn Gly Tyr Arg Trp Ile
   450 455 460
   Thr Tyr Ile Ala Asn Ser Gly Gln Arg Arg Tyr Ile Ala Thr Gly Glu
   465 470 475 480
   Val Asp Ile Ala Gly Asn Arg Ile Ser Ser Phe Gly Lys Phe Ser Ala
   485 490 495
   Val
<210> 13
   <211> 497
   <212> PRT
   <213> artificial
<220>
   <223> Polypeptide comprising substitutions at L56, L57, E164, R168, Y201, Q171, Q175, S384
<400> 13
   Met Ala Ser Ile Ile Met Glu Val Ala Thr Met Gln Ala Lys Leu Thr
   1 5 10 15
   Lys Lys Glu Phe Ile Glu Trp Leu Lys Thr Ser Glu Gly Lys Gln Phe
   20 25 30
   Asn Val Asp Leu Trp Tyr Gly Phe Gln Cys Phe Asp Tyr Ala Asn Ala
   35 40 45
   Gly Trp Lys Val Leu Phe Gly His Thr Leu Lys Gly Leu Gly Ala Lys
   50 55 60
   Asp Ile Pro Phe Ala Asn Asn Phe Asp Gly Leu Ala Thr Val Tyr Gln
   65 70 75 80
   Asn Thr Pro Asp Phe Leu Ala Gln Pro Gly Asp Met Val Val Phe Gly
   85 90 95
   Ser Asn Tyr Gly Ala Gly Tyr Gly His Val Ala Trp Val Ile Glu Ala
   100 105 110
   Thr Leu Asp Tyr Ile Ile Val Tyr Glu Gln Asn Trp Leu Gly Gly Gly
   115 120 125
   Trp Thr Asp Arg Ile Glu Gln Pro Gly Trp Gly Trp Glu Lys Val Thr
   130 135 140
   Arg Arg Gln His Ala Tyr Asp Phe Pro Met Trp Phe Ile Arg Pro Asn
   145 150 155 160
   Phe Lys Ser Ala Thr Ala Pro Ala Ser Ile Ala Ser Pro Thr Ala Ala
   165 170 175
   Ser Lys Lys Glu Thr Ala Lys Pro Gln Pro Lys Ala Val Glu Leu Lys
   180 185 190
   Ile Ile Lys Asp Val Val Lys Gly His Asp Leu Pro Lys Arg Gly Gly
   195 200 205
   Asn Pro Lys Gly Ile Val Ile His Asn Asp Ala Gly Ser Lys Gly Ala
   210 215 220
   Thr Ala Glu Ala Tyr Arg Asn Gly Leu Val Asn Ala Pro Ser Ser Arg
   225 230 235 240
   Leu Glu Ala Gly Ile Ala His Ser Tyr Val Ser Gly Asn Thr Val Trp
   245 250 255
   Gln Ala Leu Asp Glu Ser Gln Val Gly Trp His Thr Ala Asn Gln Leu
   260 265 270
   Gly Asn Lys Tyr Tyr Tyr Gly Ile Glu Val Cys Gln Ser Met Gly Ala
   275 280 285
   Asp Asn Ala Thr Phe Leu Lys Asn Glu Gln Ala Thr Phe Gln Glu Cys
   290 295 300
   Ala Arg Leu Leu Lys Lys Trp Gly Leu Pro Ala Asn Arg Asn Thr Ile
   305 310 315 320
   Arg Leu His Asn Glu Phe Thr Ser Thr Ser Cys Pro His Arg Ser Ser
   325 330 335
   Val Leu His Thr Gly Phe Asp Pro Val Thr Arg Gly Leu Leu Pro Glu
   340 345 350
   Asp Lys Arg Leu Gln Leu Lys Asp Tyr Phe Ile Lys Gln Ile Arg Ala
   355 360 365
   Tyr Met Asp Gly Lys Ile Pro Val Ala Thr Val Ser Asn Glu Ser Ala
   370 375 380
   Ala Ser Ser Asn Thr Val Lys Pro Val Ala Glu Leu Met Pro Pro Val
   385 390 395 400
   Pro Ala Gly Tyr Thr Leu Asp Lys Asn Asn Val Pro Tyr Lys Lys Glu
   405 410 415
   Gln Gly Asn Tyr Thr Val Ala Asn Val Lys Gly Asn Asn Val Arg Asp
   420 425 430
   Gly Tyr Ser Thr Asn Ser Arg Ile Thr Gly Val Leu Pro Asn Asn Thr
   435 440 445
   Thr Ile Thr Tyr Asp Gly Ala Tyr Cys Ile Asn Gly Tyr Arg Trp Ile
   450 455 460
   Thr Tyr Ile Ala Asn Ser Gly Gln Arg Arg Tyr Ile Ala Thr Gly Glu
   465 470 475 480
   Val Asp Ile Ala Gly Asn Arg Ile Ser Ser Phe Gly Lys Phe Ser Ala
   485 490 495
   Val
<210> 14
   <211> 497
   <212> PRT
   <213> artificial
<220>
   <223> Polypeptide comprising substitution at R168
<400> 14
   Met Ala Ser Ile Ile Met Glu Val Ala Thr Met Gln Ala Lys Leu Thr
   1 5 10 15
   Lys Lys Glu Phe Ile Glu Trp Leu Lys Thr Ser Glu Gly Lys Gln Phe
   20 25 30
   Asn Val Asp Leu Trp Tyr Gly Phe Gln Cys Phe Asp Tyr Ala Asn Ala
   35 40 45
   Gly Trp Lys Val Leu Phe Gly Leu Leu Leu Lys Gly Leu Gly Ala Lys
   50 55 60
   Asp Ile Pro Phe Ala Asn Asn Phe Asp Gly Leu Ala Thr Val Tyr Gln
   65 70 75 80
   Asn Thr Pro Asp Phe Leu Ala Gln Pro Gly Asp Met Val Val Phe Gly
   85 90 95
   Ser Asn Tyr Gly Ala Gly Tyr Gly His Val Ala Trp Val Ile Glu Ala
   100 105 110
   Thr Leu Asp Tyr Ile Ile Val Tyr Glu Gln Asn Trp Leu Gly Gly Gly
   115 120 125
   Trp Thr Asp Arg Ile Glu Gln Pro Gly Trp Gly Trp Glu Lys Val Thr
   130 135 140
   Arg Arg Gln His Ala Tyr Asp Phe Pro Met Trp Phe Ile Arg Pro Asn
   145 150 155 160
   Phe Lys Ser Glu Thr Ala Pro Ala Ser Ile Gln Ser Pro Thr Gln Ala
   165 170 175
   Ser Lys Lys Glu Thr Ala Lys Pro Gln Pro Lys Ala Val Glu Leu Lys
   180 185 190
   Ile Ile Lys Asp Val Val Lys Gly Tyr Asp Leu Pro Lys Arg Gly Gly
   195 200 205
   Asn Pro Lys Gly Ile Val Ile His Asn Asp Ala Gly Ser Lys Gly Ala
   210 215 220
   Thr Ala Glu Ala Tyr Arg Asn Gly Leu Val Asn Ala Pro Ser Ser Arg
   225 230 235 240
   Leu Glu Ala Gly Ile Ala His Ser Tyr Val Ser Gly Asn Thr Val Trp
   245 250 255
   Gln Ala Leu Asp Glu Ser Gln Val Gly Trp His Thr Ala Asn Gln Leu
   260 265 270
   Gly Asn Lys Tyr Tyr Tyr Gly Ile Glu Val Cys Gln Ser Met Gly Ala
   275 280 285
   Asp Asn Ala Thr Phe Leu Lys Asn Glu Gln Ala Thr Phe Gln Glu Cys
   290 295 300
   Ala Arg Leu Leu Lys Lys Trp Gly Leu Pro Ala Asn Arg Asn Thr Ile
   305 310 315 320
   Arg Leu His Asn Glu Phe Thr Ser Thr Ser Cys Pro His Arg Ser Ser
   325 330 335
   Val Leu His Thr Gly Phe Asp Pro Val Thr Arg Gly Leu Leu Pro Glu
   340 345 350
   Asp Lys Arg Leu Gln Leu Lys Asp Tyr Phe Ile Lys Gln Ile Arg Ala
   355 360 365
   Tyr Met Asp Gly Lys Ile Pro Val Ala Thr Val Ser Asn Glu Ser Ser
   370 375 380
   Ala Ser Ser Asn Thr Val Lys Pro Val Ala Glu Leu Met Pro Pro Val
   385 390 395 400
   Pro Ala Gly Tyr Thr Leu Asp Lys Asn Asn Val Pro Tyr Lys Lys Glu
   405 410 415
   Gln Gly Asn Tyr Thr Val Ala Asn Val Lys Gly Asn Asn Val Arg Asp
   420 425 430
   Gly Tyr Ser Thr Asn Ser Arg Ile Thr Gly Val Leu Pro Asn Asn Thr
   435 440 445
   Thr Ile Thr Tyr Asp Gly Ala Tyr Cys Ile Asn Gly Tyr Arg Trp Ile
   450 455 460
   Thr Tyr Ile Ala Asn Ser Gly Gln Arg Arg Tyr Ile Ala Thr Gly Glu
   465 470 475 480
   Val Asp Ile Ala Gly Asn Arg Ile Ser Ser Phe Gly Lys Phe Ser Ala
   485 490 495
   Val
<210> 15
   <211> 497
   <212> PRT
   <213> artificial
<220>
   <223> Polypetide comprising substitution at E163 and R167
<400> 15
   Met Ala Ser Ile Ile Met Glu Val Ala Thr Met Gln Ala Lys Leu Thr
   1 5 10 15
   Lys Lys Glu Phe Ile Glu Trp Leu Lys Thr Ser Glu Gly Lys Gln Phe
   20 25 30
   Asn Val Asp Leu Trp Tyr Gly Phe Gln Cys Phe Asp Tyr Ala Asn Ala
   35 40 45
   Gly Trp Lys Val Leu Phe Gly Leu Leu Leu Lys Gly Leu Gly Ala Lys
   50 55 60
   Asp Ile Pro Phe Ala Asn Asn Phe Asp Gly Leu Ala Thr Val Tyr Gln
   65 70 75 80
   Asn Thr Pro Asp Phe Leu Ala Gln Pro Gly Asp Met Val Val Phe Gly
   85 90 95
   Ser Asn Tyr Gly Ala Gly Tyr Gly His Val Ala Trp Val Ile Glu Ala
   100 105 110
   Thr Leu Asp Tyr Ile Ile Val Tyr Glu Gln Asn Trp Leu Gly Gly Gly
   115 120 125
   Trp Thr Asp Arg Ile Glu Gln Pro Gly Trp Gly Trp Glu Lys Val Thr
   130 135 140
   Arg Arg Gln His Ala Tyr Asp Phe Pro Met Trp Phe Ile Arg Pro Asn
   145 150 155 160
   Phe Lys Ser Ala Thr Ala Pro Ala Ser Ile Gln Ser Pro Thr Gln Ala
   165 170 175
   Ser Lys Lys Glu Thr Ala Lys Pro Gln Pro Lys Ala Val Glu Leu Lys
   180 185 190
   Ile Ile Lys Asp Val Val Lys Gly Tyr Asp Leu Pro Lys Arg Gly Gly
   195 200 205
   Asn Pro Lys Gly Ile Val Ile His Asn Asp Ala Gly Ser Lys Gly Ala
   210 215 220
   Thr Ala Glu Ala Tyr Arg Asn Gly Leu Val Asn Ala Pro Ser Ser Arg
   225 230 235 240
   Leu Glu Ala Gly Ile Ala His Ser Tyr Val Ser Gly Asn Thr Val Trp
   245 250 255
   Gln Ala Leu Asp Glu Ser Gln Val Gly Trp His Thr Ala Asn Gln Leu
               260                 265                 270
   Gly Asn Lys Tyr Tyr Tyr Gly Ile Glu Val Cys Gln Ser Met Gly Ala
           275                 280                 285
   Asp Asn Ala Thr Phe Leu Lys Asn Glu Gln Ala Thr Phe Gln Glu Cys
       290                 295                 300
   Ala Arg Leu Leu Lys Lys Trp Gly Leu Pro Ala Asn Arg Asn Thr Ile
   305                 310                 315                 320
   Arg Leu His Asn Glu Phe Thr Ser Thr Ser Cys Pro His Arg Ser Ser
                   325                 330                 335
   Val Leu His Thr Gly Phe Asp Pro Val Thr Arg Gly Leu Leu Pro Glu
               340                 345                 350
   Asp Lys Arg Leu Gln Leu Lys Asp Tyr Phe Ile Lys Gln Ile Arg Ala
           355                 360                 365
   Tyr Met Asp Gly Lys Ile Pro Val Ala Thr Val Ser Asn Glu Ser Ser
       370                 375                 380
   Ala Ser Ser Asn Thr Val Lys Pro Val Ala Glu Leu Met Pro Pro Val
   385                 390                 395                 400
   Pro Ala Gly Tyr Thr Leu Asp Lys Asn Asn Val Pro Tyr Lys Lys Glu
                   405                 410                 415
   Gln Gly Asn Tyr Thr Val Ala Asn Val Lys Gly Asn Asn Val Arg Asp
               420                 425                 430
   Gly Tyr Ser Thr Asn Ser Arg Ile Thr Gly Val Leu Pro Asn Asn Thr
           435                 440                 445
   Thr Ile Thr Tyr Asp Gly Ala Tyr Cys Ile Asn Gly Tyr Arg Trp Ile
       450                 455                 460
   Thr Tyr Ile Ala Asn Ser Gly Gln Arg Arg Tyr Ile Ala Thr Gly Glu
   465                 470                 475                 480
   Val Asp Ile Ala Gly Asn Arg Ile Ser Ser Phe Gly Lys Phe Ser Ala
                   485                 490                 495
   Val
<210> 16
   <211> 497
   <212> PRT
   <213> artificial
<220>
   <223> Polypetide comprising substitution at E163, R167, E179, E189
<400> 16
   Met Ala Ser Ile Ile Met Glu Val Ala Thr Met Gln Ala Lys Leu Thr
   1               5                   10                  15
   Lys Lys Glu Phe Ile Glu Trp Leu Lys Thr Ser Glu Gly Lys Gln Phe
               20                  25                  30
   Asn Val Asp Leu Trp Tyr Gly Phe Gln Cys Phe Asp Tyr Ala Asn Ala
           35                  40                  45
   Gly Trp Lys Val Leu Phe Gly Leu Leu Leu Lys Gly Leu Gly Ala Lys
       50                  55                  60
   Asp Ile Pro Phe Ala Asn Asn Phe Asp Gly Leu Ala Thr Val Tyr Gln
   65                  70                  75                  80
   Asn Thr Pro Asp Phe Leu Ala Gln Pro Gly Asp Met Val Val Phe Gly
                   85                  90                  95
   Ser Asn Tyr Gly Ala Gly Tyr Gly His Val Ala Trp Val Ile Glu Ala
               100                 105                 110
   Thr Leu Asp Tyr Ile Ile Val Tyr Glu Gln Asn Trp Leu Gly Gly Gly
           115                 120                 125
   Trp Thr Asp Arg Ile Glu Gln Pro Gly Trp Gly Trp Glu Lys Val Thr
       130                 135                 140
   Arg Arg Gln His Ala Tyr Asp Phe Pro Met Trp Phe Ile Arg Pro Asn
   145                 150                 155                 160
   Phe Lys Ser Gln Thr Ala Pro Ala Ser Ile Gln Ser Pro Thr Gln Ala
                   165                 170                 175
   Ser Lys Lys Ala Thr Ala Lys Pro Gln Pro Lys Ala Val Gln Leu Lys
               180                 185                 190
   Ile Ile Lys Asp Val Val Lys Gly Tyr Asp Leu Pro Lys Arg Gly Gly
           195                 200                 205
   Asn Pro Lys Gly Ile Val Ile His Asn Asp Ala Gly Ser Lys Gly Ala
       210                 215                 220
   Thr Ala Glu Ala Tyr Arg Asn Gly Leu Val Asn Ala Pro Ser Ser Arg
   225 230 235 240
   Leu Glu Ala Gly Ile Ala His Ser Tyr Val Ser Gly Asn Thr Val Trp
   245 250 255
   Gln Ala Leu Asp Glu Ser Gln Val Gly Trp His Thr Ala Asn Gln Leu
   260 265 270
   Gly Asn Lys Tyr Tyr Tyr Gly Ile Glu Val Cys Gln Ser Met Gly Ala
   275 280 285
   Asp Asn Ala Thr Phe Leu Lys Asn Glu Gln Ala Thr Phe Gln Glu Cys
   290 295 300
   Ala Arg Leu Leu Lys Lys Trp Gly Leu Pro Ala Asn Arg Asn Thr Ile
   305 310 315 320
   Arg Leu His Asn Glu Phe Thr Ser Thr Ser Cys Pro His Arg Ser Ser
   325 330 335
   Val Leu His Thr Gly Phe Asp Pro Val Thr Arg Gly Leu Leu Pro Glu
   340 345 350
   Asp Lys Arg Leu Gln Leu Lys Asp Tyr Phe Ile Lys Gln Ile Arg Ala
   355 360 365
   Tyr Met Asp Gly Lys Ile Pro Val Ala Thr Val Ser Asn Glu Ser Ser
   370 375 380
   Ala Ser Ser Asn Thr Val Lys Pro Val Ala Glu Leu Met Pro Pro Val
   385 390 395 400
   Pro Ala Gly Tyr Thr Leu Asp Lys Asn Asn Val Pro Tyr Lys Lys Glu
   405 410 415
   Gln Gly Asn Tyr Thr Val Ala Asn Val Lys Gly Asn Asn Val Arg Asp
   420 425 430
   Gly Tyr Ser Thr Asn Ser Arg Ile Thr Gly Val Leu Pro Asn Asn Thr
   435 440 445
   Thr Ile Thr Tyr Asp Gly Ala Tyr Cys Ile Asn Gly Tyr Arg Trp Ile
   450 455 460
   Thr Tyr Ile Ala Asn Ser Gly Gln Arg Arg Tyr Ile Ala Thr Gly Glu
   465 470 475 480
   Val Asp Ile Ala Gly Asn Arg Ile Ser Ser Phe Gly Lys Phe Ser Ala
   485 490 495
   Val
<210> 17
   <211> 497
   <212> PRT
   <213> artificial
<220>
   <223> Polypetide comprising substitution at E163, R167
<400> 17
   Met Ala Ser Ile Ile Met Glu Val Ala Thr Met Gln Ala Lys Leu Thr
   1 5 10 15
   Lys Lys Glu Phe Ile Glu Trp Leu Lys Thr Ser Glu Gly Lys Gln Phe
   20 25 30
   Asn Val Asp Leu Trp Tyr Gly Phe Gln Cys Phe Asp Tyr Ala Asn Ala
   35 40 45
   Gly Trp Lys Val Leu Phe Gly Leu Leu Leu Lys Gly Leu Gly Ala Lys
   50 55 60
   Asp Ile Pro Phe Ala Asn Asn Phe Asp Gly Leu Ala Thr Val Tyr Gln
   65 70 75 80
   Asn Thr Pro Asp Phe Leu Ala Gln Pro Gly Asp Met Val Val Phe Gly
   85 90 95
   Ser Asn Tyr Gly Ala Gly Tyr Gly His Val Ala Trp Val Ile Glu Ala
   100 105 110
   Thr Leu Asp Tyr Ile Ile Val Tyr Glu Gln Asn Trp Leu Gly Gly Gly
   115 120 125
   Trp Thr Asp Arg Ile Glu Gln Pro Gly Trp Gly Trp Glu Lys Val Thr
   130 135 140
   Arg Arg Gln His Ala Tyr Asp Phe Pro Met Trp Phe Ile Arg Pro Asn
   145 150 155 160
   Phe Lys Ser Gln Thr Ala Pro Ala Ser Ile Gln Ser Pro Thr Gln Ala
   165 170 175
   Ser Lys Lys Glu Thr Ala Lys Pro Gln Pro Lys Ala Val Glu Leu Lys
   180 185 190
   Ile Ile Lys Asp Val Val Lys Gly Tyr Asp Leu Pro Lys Arg Gly Gly
   195 200 205
   Asn Pro Lys Gly Ile Val Ile His Asn Asp Ala Gly Ser Lys Gly Ala
   210 215 220
   Thr Ala Glu Ala Tyr Arg Asn Gly Leu Val Asn Ala Pro Ser Ser Arg
   225 230 235 240
   Leu Glu Ala Gly Ile Ala His Ser Tyr Val Ser Gly Asn Thr Val Trp
   245 250 255
   Gln Ala Leu Asp Glu Ser Gln Val Gly Trp His Thr Ala Asn Gln Leu
   260 265 270
   Gly Asn Lys Tyr Tyr Tyr Gly Ile Glu Val Cys Gln Ser Met Gly Ala
   275 280 285
   Asp Asn Ala Thr Phe Leu Lys Asn Glu Gln Ala Thr Phe Gln Glu Cys
   290 295 300
   Ala Arg Leu Leu Lys Lys Trp Gly Leu Pro Ala Asn Arg Asn Thr Ile
   305 310 315 320
   Arg Leu His Asn Glu Phe Thr Ser Thr Ser Cys Pro His Arg Ser Ser
   325 330 335
   Val Leu His Thr Gly Phe Asp Pro Val Thr Arg Gly Leu Leu Pro Glu
   340 345 350
   Asp Lys Arg Leu Gln Leu Lys Asp Tyr Phe Ile Lys Gln Ile Arg Ala
   355 360 365
   Tyr Met Asp Gly Lys Ile Pro Val Ala Thr Val Ser Asn Glu Ser Ser
   370 375 380
   Ala Ser Ser Asn Thr Val Lys Pro Val Ala Glu Leu Met Pro Pro Val
   385 390 395 400
   Pro Ala Gly Tyr Thr Leu Asp Lys Asn Asn Val Pro Tyr Lys Lys Glu
   405 410 415
   Gln Gly Asn Tyr Thr Val Ala Asn Val Lys Gly Asn Asn Val Arg Asp
   420 425 430
   Gly Tyr Ser Thr Asn Ser Arg Ile Thr Gly Val Leu Pro Asn Asn Thr
   435 440 445
   Thr Ile Thr Tyr Asp Gly Ala Tyr Cys Ile Asn Gly Tyr Arg Trp Ile
   450 455 460
   Thr Tyr Ile Ala Asn Ser Gly Gln Arg Arg Tyr Ile Ala Thr Gly Glu
   465 470 475 480
   Val Asp Ile Ala Gly Asn Arg Ile Ser Ser Phe Gly Lys Phe Ser Ala
   485 490 495
   Val
<210> 18
   <211> 496
   <212> PRT
   <213> artificial
<220>
   <223> CHAP-AMIpitti26-CBDUSA
<400> 18
   Met Ser Ile Ile Met Glu Val Ala Thr Met Gln Ala Lys Leu Thr Lys
   1 5 10 15
   Lys Glu Phe Ile Glu Trp Leu Lys Thr Ser Glu Gly Lys Gln Phe Asn
   20 25 30
   Val Asp Leu Trp Tyr Gly Phe Gln Cys Phe Asp Tyr Ala Asn Ala Gly
   35 40 45
   Trp Lys Val Leu Phe Gly Leu Leu Leu Lys Gly Leu Gly Ala Lys Asp
   50 55 60
   Ile Pro Phe Ala Asn Asn Phe Asp Gly Leu Ala Thr Val Tyr Gln Asn
   65 70 75 80
   Thr Pro Asp Phe Leu Ala Gln Pro Gly Asp Met Val Val Phe Gly Ser
   85 90 95
   Asn Tyr Gly Ala Gly Tyr Gly His Val Ala Trp Val Ile Glu Ala Thr
   100 105 110
   Leu Asp Tyr Ile Ile Val Tyr Glu Gln Asn Trp Leu Gly Gly Gly Trp
   115 120 125
   Thr Asp Arg Ile Glu Gln Pro Gly Trp Gly Trp Glu Lys Val Thr Arg
   130 135 140
   Arg Gln His Ala Tyr Asp Phe Pro Met Trp Phe Ile Arg Pro Asn Phe
   145 150 155 160
   Lys Ser Glu Thr Ala Pro Arg Ser Ile Gln Ser Pro Thr Gln Ala Ser
   165 170 175
   Lys Lys Glu Thr Ala Lys Pro Gln Pro Lys Ala Val Glu Leu Lys Ile
   180 185 190
   Ile Lys Asp Val Val Lys Gly Tyr Asp Leu Pro Lys Arg Gly Gly Asn
   195 200 205
   Pro Lys Gly Ile Val Ile His Asn Asp Ala Gly Ser Lys Gly Ala Thr
   210 215 220
   Ala Glu Ala Tyr Arg Asn Gly Leu Val Asn Ala Pro Ser Ser Arg Leu
   225 230 235 240
   Glu Ala Gly Ile Ala His Ser Tyr Val Ser Gly Asn Thr Val Trp Gln
   245 250 255
   Ala Leu Asp Glu Ser Gln Val Gly Trp His Thr Ala Asn Gln Leu Gly
   260 265 270
   Asn Lys Tyr Tyr Tyr Gly Ile Glu Val Cys Gln Ser Met Gly Ala Asp
   275 280 285
   Asn Ala Thr Phe Leu Lys Asn Glu Gln Ala Thr Phe Gln Glu Cys Ala
   290 295 300
   Arg Leu Leu Lys Lys Trp Gly Leu Pro Ala Asn Arg Asn Thr Ile Arg
   305 310 315 320
   Leu His Asn Glu Phe Thr Ser Thr Ser Cys Pro His Arg Ser Ser Val
   325 330 335
   Leu His Thr Gly Phe Asp Pro Val Thr Arg Gly Leu Leu Pro Glu Asp
   340 345 350
   Lys Arg Leu Gln Leu Lys Asp Tyr Phe Ile Lys Gln Ile Arg Ala Tyr
   355 360 365
   Met Asp Gly Lys Ile Pro Val Ala Thr Val Ser Asn Glu Ser Ser Ala
   370 375 380
   Ser Ser Asn Thr Val Lys Pro Val Ala Glu Leu Met Pro Pro Val Pro
   385 390 395 400
   Ala Gly Tyr Thr Leu Asp Lys Asn Asn Val Pro Tyr Lys Lys Glu Gln
   405 410 415
   Gly Asn Tyr Thr Val Ala Asn Val Lys Gly Asn Asn Val Arg Asp Gly
   420 425 430
   Tyr Ser Thr Asn Ser Arg Ile Thr Gly Val Leu Pro Asn Asn Thr Thr
   435 440 445
   Ile Thr Tyr Asp Gly Ala Tyr Cys Ile Asn Gly Tyr Arg Trp Ile Thr
   450 455 460
   Tyr Ile Ala Asn Ser Gly Gln Arg Arg Tyr Ile Ala Thr Gly Glu Val
   465 470 475 480
   Asp Ile Ala Gly Asn Arg Ile Ser Ser Phe Gly Lys Phe Ser Ala Val
   485 490 495
<210> 19
   <211> 497
   <212> PRT
   <213> artificial
<220>
   <223> CHAP-AMIpitti26-CBDUSA-Add2
<400> 19
   Met Ala Ser Ile Ile Met Glu Val Ala Thr Met Gln Ala Lys Leu Thr
   1 5 10 15
   Lys Lys Glu Phe Ile Glu Trp Leu Lys Thr Ser Glu Gly Lys Gln Phe
   20 25 30
   Asn Val Asp Leu Trp Tyr Gly Phe Gln Cys Phe Asp Tyr Ala Asn Ala
   35 40 45
   Gly Trp Lys Val Leu Phe Gly Leu Leu Leu Lys Gly Leu Gly Ala Lys
   50 55 60
   Asp Ile Pro Phe Ala Asn Asn Phe Asp Gly Leu Ala Thr Val Tyr Gln
   65 70 75 80
   Asn Thr Pro Asp Phe Leu Ala Gln Pro Gly Asp Met Val Val Phe Gly
   85 90 95
   Ser Asn Tyr Gly Ala Gly Tyr Gly His Val Ala Trp Val Ile Glu Ala
   100 105 110
   Thr Leu Asp Tyr Ile Ile Val Tyr Glu Gln Asn Trp Leu Gly Gly Gly
   115 120 125
   Trp Thr Asp Arg Ile Glu Gln Pro Gly Trp Gly Trp Glu Lys Val Thr
   130 135 140
   Arg Arg Gln His Ala Tyr Asp Phe Pro Met Trp Phe Ile Arg Pro Asn
   145 150 155 160
   Phe Lys Ser Glu Thr Ala Pro Arg Ser Ile Gln Ser Pro Thr Gln Ala
   165 170 175
   Ser Lys Lys Glu Thr Ala Lys Pro Gln Pro Lys Ala Val Glu Leu Lys
   180 185 190
   Ile Ile Lys Asp Val Val Lys Gly Tyr Asp Leu Pro Lys Arg Gly Gly
   195 200 205
   Asn Pro Lys Gly Ile Val Ile His Asn Asp Ala Gly Ser Lys Gly Ala
   210 215 220
   Thr Ala Glu Ala Tyr Arg Asn Gly Leu Val Asn Ala Pro Ser Ser Arg
   225 230 235 240
   Leu Glu Ala Gly Ile Ala His Ser Tyr Val Ser Gly Asn Thr Val Trp
   245 250 255
   Gln Ala Leu Asp Glu Ser Gln Val Gly Trp His Thr Ala Asn Gln Leu
   260 265 270
   Gly Asn Lys Tyr Tyr Tyr Gly Ile Glu Val Cys Gln Ser Met Gly Ala
   275 280 285
   Asp Asn Ala Thr Phe Leu Lys Asn Glu Gln Ala Thr Phe Gln Glu Cys
   290 295 300
   Ala Arg Leu Leu Lys Lys Trp Gly Leu Pro Ala Asn Arg Asn Thr Ile
   305 310 315 320
   Arg Leu His Asn Glu Phe Thr Ser Thr Ser Cys Pro His Arg Ser Ser
   325 330 335
   Val Leu His Thr Gly Phe Asp Pro Val Thr Arg Gly Leu Leu Pro Glu
   340 345 350
   Asp Lys Arg Leu Gln Leu Lys Asp Tyr Phe Ile Lys Gln Ile Arg Ala
   355 360 365
   Tyr Met Asp Gly Lys Ile Pro Val Ala Thr Val Ser Asn Glu Ser Ser
   370 375 380
   Ala Ser Ser Asn Thr Val Lys Pro Val Ala Glu Leu Met Pro Pro Val
   385 390 395 400
   Pro Ala Gly Tyr Thr Leu Asp Lys Asn Asn Val Pro Tyr Lys Lys Glu
   405 410 415
   Gln Gly Asn Tyr Thr Val Ala Asn Val Lys Gly Asn Asn Val Arg Asp
   420 425 430
   Gly Tyr Ser Thr Asn Ser Arg Ile Thr Gly Val Leu Pro Asn Asn Thr
   435 440 445
   Thr Ile Thr Tyr Asp Gly Ala Tyr Cys Ile Asn Gly Tyr Arg Trp Ile
   450 455 460
   Thr Tyr Ile Ala Asn Ser Gly Gln Arg Arg Tyr Ile Ala Thr Gly Glu
   465 470 475 480
   Val Asp Ile Ala Gly Asn Arg Ile Ser Ser Phe Gly Lys Phe Ser Ala
   485 490 495
   Val
<210> 20
   <211> 393
   <212> PRT
   <213> artificial
<220>
   <223> CHAP-AMIpitti26
<400> 20
   Met Ser Ile Ile Met Glu Val Ala Thr Met Gln Ala Lys Leu Thr Lys
   1 5 10 15
   Lys Glu Phe Ile Glu Trp Leu Lys Thr Ser Glu Gly Lys Gln Phe Asn
   20 25 30
   Val Asp Leu Trp Tyr Gly Phe Gln Cys Phe Asp Tyr Ala Asn Ala Gly
   35 40 45
   Trp Lys Val Leu Phe Gly Leu Leu Leu Lys Gly Leu Gly Ala Lys Asp
   50 55 60
   Ile Pro Phe Ala Asn Asn Phe Asp Gly Leu Ala Thr Val Tyr Gln Asn
   65 70 75 80
   Thr Pro Asp Phe Leu Ala Gln Pro Gly Asp Met Val Val Phe Gly Ser
   85 90 95
   Asn Tyr Gly Ala Gly Tyr Gly His Val Ala Trp Val Ile Glu Ala Thr
   100 105 110
   Leu Asp Tyr Ile Ile Val Tyr Glu Gln Asn Trp Leu Gly Gly Gly Trp
           115                 120                 125
   Thr Asp Arg Ile Glu Gln Pro Gly Trp Gly Trp Glu Lys Val Thr Arg
       130                 135                 140
   Arg Gln His Ala Tyr Asp Phe Pro Met Trp Phe Ile Arg Pro Asn Phe
   145                 150                 155                 160
   Lys Ser Glu Thr Ala Pro Arg Ser Ile Gln Ser Pro Thr Gln Ala Ser
                   165                 170                 175
   Lys Lys Glu Thr Ala Lys Pro Gln Pro Lys Ala Val Glu Leu Lys Ile
               180                 185                 190
   Ile Lys Asp Val Val Lys Gly Tyr Asp Leu Pro Lys Arg Gly Gly Asn
           195                 200                 205
   Pro Lys Gly Ile Val Ile His Asn Asp Ala Gly Ser Lys Gly Ala Thr
       210                 215                 220
   Ala Glu Ala Tyr Arg Asn Gly Leu Val Asn Ala Pro Ser Ser Arg Leu
   225                 230                 235                 240
   Glu Ala Gly Ile Ala His Ser Tyr Val Ser Gly Asn Thr Val Trp Gln
                   245                 250                 255
   Ala Leu Asp Glu Ser Gln Val Gly Trp His Thr Ala Asn Gln Leu Gly
               260                 265                 270
   Asn Lys Tyr Tyr Tyr Gly Ile Glu Val Cys Gln Ser Met Gly Ala Asp
           275                 280                 285
   Asn Ala Thr Phe Leu Lys Asn Glu Gln Ala Thr Phe Gln Glu Cys Ala
       290                 295                 300
   Arg Leu Leu Lys Lys Trp Gly Leu Pro Ala Asn Arg Asn Thr Ile Arg
   305                 310                 315                 320
   Leu His Asn Glu Phe Thr Ser Thr Ser Cys Pro His Arg Ser Ser Val
                   325                 330                 335
   Leu His Thr Gly Phe Asp Pro Val Thr Arg Gly Leu Leu Pro Glu Asp
               340                 345                 350
   Lys Arg Leu Gln Leu Lys Asp Tyr Phe Ile Lys Gln Ile Arg Ala Tyr
           355                 360                 365
   Met Asp Gly Lys Ile Pro Val Ala Thr Val Ser Asn Glu Ser Ser Ala
   370 375 380
   Ser Ser Asn Thr Val Lys Pro Val Ala
   385 390
<210> 21
   <211> 341
   <212> PRT
   <213> Bacteriophage A511
<400> 21
   Met Val Lys Tyr Thr Val Glu Asn Lys Ile Ile Ala Gly Leu Pro Lys
   1 5 10 15
   Gly Lys Leu Lys Gly Ala Asn Phe Val Ile Ala His Glu Thr Ala Asn
   20 25 30
   Ser Lys Ser Thr Ile Asp Asn Glu Val Ser Tyr Met Thr Arg Asn Trp
   35 40 45
   Lys Asn Ala Phe Val Thr His Phe Val Gly Gly Gly Gly Arg Val Val
   50 55 60
   Gln Val Ala Asn Val Asn Tyr Val Ser Trp Gly Ala Gly Gln Tyr Ala
   65 70 75 80
   Asn Ser Tyr Ser Tyr Ala Gln Val Glu Leu Cys Arg Thr Ser Asn Ala
   85 90 95
   Thr Thr Phe Lys Lys Asp Tyr Glu Val Tyr Cys Gln Leu Leu Val Asp
   100 105 110
   Leu Ala Lys Lys Ala Gly Ile Pro Ile Thr Leu Asp Ser Gly Ser Lys
   115 120 125
   Thr Ser Asp Lys Gly Ile Lys Ser His Lys Trp Val Ala Asp Lys Leu
   130 135 140
   Gly Gly Thr Thr His Gln Asp Pro Tyr Ala Tyr Leu Ser Ser Trp Gly
   145 150 155 160
   Ile Ser Lys Ala Gln Phe Ala Ser Asp Leu Ala Lys Val Ser Gly Gly
   165 170 175
   Gly Asn Thr Gly Thr Ala Pro Ala Lys Pro Ser Thr Pro Ala Pro Lys
   180 185 190
   Pro Ser Thr Pro Ser Thr Asn Leu Asp Lys Leu Gly Leu Val Asp Tyr
   195 200 205
   Met Asn Ala Lys Lys Met Asp Ser Ser Tyr Ser Asn Arg Asp Lys Leu
   210 215 220
   Ala Lys Gln Tyr Gly Ile Ala Asn Tyr Ser Gly Thr Ala Ser Gln Asn
   225 230 235 240
   Thr Thr Leu Leu Ser Lys Ile Lys Gly Gly Ala Pro Lys Pro Ser Thr
   245 250 255
   Pro Ala Pro Lys Pro Ser Thr Ser Thr Ala Lys Lys Ile Tyr Phe Pro
   260 265 270
   Pro Asn Lys Gly Asn Trp Ser Val Tyr Pro Thr Asn Lys Ala Pro Val
   275 280 285
   Lys Ala Asn Ala Ile Gly Ala Ile Asn Pro Thr Lys Phe Gly Gly Leu
   290 295 300
   Thr Tyr Thr Ile Gln Lys Asp Arg Gly Asn Gly Val Tyr Glu Ile Gln
   305 310 315 320
   Thr Asp Gln Phe Gly Arg Val Gln Val Tyr Gly Ala Pro Ser Thr Gly
   325 330 335
   Ala Val Ile Lys Lys
   340
<210> 22
   <211> 497
   <212> PRT
   <213> artificial
<220>
   <223> Polypeptide comprising substitutions at L56, L57, E164, R168, Y201
<400> 22
   Met Ala Ser Ile Ile Met Glu Val Ala Thr Met Gln Ala Lys Leu Thr
   1 5 10 15
   Lys Lys Glu Phe Ile Glu Trp Leu Lys Thr Ser Glu Gly Lys Gln Phe
   20 25 30
   Asn Val Asp Leu Trp Tyr Gly Phe Gln Cys Phe Asp Tyr Ala Asn Ala
   35 40 45
   Gly Trp Lys Val Leu Phe Gly His Thr Leu Lys Gly Leu Gly Ala Lys
   50 55 60
   Asp Ile Pro Phe Ala Asn Asn Phe Asp Gly Leu Ala Thr Val Tyr Gln
   65 70 75 80
   Asn Thr Pro Asp Phe Leu Ala Gln Pro Gly Asp Met Val Val Phe Gly
   85 90 95
   Ser Asn Tyr Gly Ala Gly Tyr Gly His Val Ala Trp Val Ile Glu Ala
   100 105 110
   Thr Leu Asp Tyr Ile Ile Val Tyr Glu Gln Asn Trp Leu Gly Gly Gly
   115 120 125
   Trp Thr Asp Arg Ile Glu Gln Pro Gly Trp Gly Trp Glu Lys Val Thr
   130 135 140
   Arg Arg Gln His Ala Tyr Asp Phe Pro Met Trp Phe Ile Arg Pro Asn
   145 150 155 160
   Phe Lys Ser Ala Thr Ala Pro Ala Ser Ile Gln Ser Pro Thr Gln Ala
   165 170 175
   Ser Lys Lys Glu Thr Ala Lys Pro Gln Pro Lys Ala Val Glu Leu Lys
   180 185 190
   Ile Ile Lys Asp Val Val Lys Gly His Asp Leu Pro Lys Arg Gly Gly
   195 200 205
   Asn Pro Lys Gly Ile Val Ile His Asn Asp Ala Gly Ser Lys Gly Ala
   210 215 220
   Thr Ala Glu Ala Tyr Arg Asn Gly Leu Val Asn Ala Pro Ser Ser Arg
   225 230 235 240
   Leu Glu Ala Gly Ile Ala His Ser Tyr Val Ser Gly Asn Thr Val Trp
   245 250 255
   Gln Ala Leu Asp Glu Ser Gln Val Gly Trp His Thr Ala Asn Gln Leu
   260 265 270
   Gly Asn Lys Tyr Tyr Tyr Gly Ile Glu Val Cys Gln Ser Met Gly Ala
   275 280 285
   Asp Asn Ala Thr Phe Leu Lys Asn Glu Gln Ala Thr Phe Gln Glu Cys
   290 295 300
   Ala Arg Leu Leu Lys Lys Trp Gly Leu Pro Ala Asn Arg Asn Thr Ile
   305 310 315 320
   Arg Leu His Asn Glu Phe Thr Ser Thr Ser Cys Pro His Arg Ser Ser
   325 330 335
   Val Leu His Thr Gly Phe Asp Pro Val Thr Arg Gly Leu Leu Pro Glu
               340                 345                 350
   Asp Lys Arg Leu Gln Leu Lys Asp Tyr Phe Ile Lys Gln Ile Arg Ala
           355                 360                 365
   Tyr Met Asp Gly Lys Ile Pro Val Ala Thr Val Ser Asn Glu Ser Ser
       370                 375                 380
   Ala Ser Ser Asn Thr Val Lys Pro Val Ala Glu Leu Met Pro Pro Val
   385                 390                 395                 400
   Pro Ala Gly Tyr Thr Leu Asp Lys Asn Asn Val Pro Tyr Lys Lys Glu
                   405                 410                 415
   Gln Gly Asn Tyr Thr Val Ala Asn Val Lys Gly Asn Asn Val Arg Asp
               420                 425                 430
   Gly Tyr Ser Thr Asn Ser Arg Ile Thr Gly Val Leu Pro Asn Asn Thr
           435                 440                 445
   Thr Ile Thr Tyr Asp Gly Ala Tyr Cys Ile Asn Gly Tyr Arg Trp Ile
       450                 455                 460
   Thr Tyr Ile Ala Asn Ser Gly Gln Arg Arg Tyr Ile Ala Thr Gly Glu
   465                 470                 475                 480
   Val Asp Ile Ala Gly Asn Arg Ile Ser Ser Phe Gly Lys Phe Ser Ala
                   485                 490                 495
   Val